(19) Europäisches Patentamt
European Patent Office
Office européen des brevets

(11) EP 0 912 097 B1

(12) **EUROPEAN PATENT SPECIFICATION**

(45) Date of publication and mention of the grant of the patent:
**07.08.2002 Bulletin 2002/32**

(21) Application number: **97920611.7**

(22) Date of filing: **06.05.1997**

(51) Int Cl.[7]: **A01N 63/00**, A01N 63/04, A01N 59/00, A01N 43/84, A01N 37/10, C11D 3/386, C11D 3/48

(86) International application number:
**PCT/DK97/00205**

(87) International publication number:
**WO 97/42825 (20.11.1997 Gazette 1997/50)**

(54) **ANTIMICROBIAL PEROXIDASE COMPOSITIONS**

ANTIMIKROBIELLE PEROXIDASE-ZUSAMMENSETZUNGEN

COMPOSITIONS ANTIMICROBIENNES DE PEROXYDASE

(84) Designated Contracting States:
**AT BE CH DE DK ES FI FR GB GR IE IT LI NL PT SE**

(30) Priority: **09.05.1996 DK 55996**
**15.07.1996 DK 78596**

(43) Date of publication of application:
**06.05.1999 Bulletin 1999/18**

(73) Proprietor: **Novozymes A/S**
**2880 Bagsvaerd (DK)**

(72) Inventor: **JOHANSEN, Charlotte**
**DK-2880 Bagsvaerd (DK)**

(56) References cited:

**EP-A- 0 500 387**
**WO-A-92/01466**
**WO-A-93/24618**
**WO-A-94/04127**
**WO-A-94/05252**
**WO-A-94/12621**
**WO-A-95/10602**
**WO-A-95/27046**
**WO-A-96/10079**
**US-A- 4 588 586**
**US-A- 4 996 146**
**US-A- 5 227 161**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

INFORMATION FOR SEQ ID NO:1:

(i) SEQUENCE CHARACTERISTICS:

(A) LENGTH: 1306 base pairs
(B) TYPE: nucleic acid
(C) STRANDEDNESS: single
(D) TOPOLOGY: linear

(ii) MOLECULE TYPE: cDNA

(vi) ORIGINAL SOURCE:

(A) ORGANISM: Coprinus cinereus
(B) STRAIN: IFO 8371

(xi) SEQUENCE DESCRIPTION: SEQ ID NO:1:

ACTATGAAGC TCTCGCTTTT GTCCACCTTC GCTGCTGTCA TCATCGGTGC CCTCGCTCTA 60

CCCCAGGGTC CTGGAGGAGG CGGGTCAGTC ACTTGCCCCG GTGGACAGTC CACTTCGAAC 120

AGCCAGTGCT GCGTCTGGTT CGACGTTCTA GACGATCTTC AGACCAACTT CTACCAAGGG 180

TCCAAGTGTG AGAGCCCTGT TCGCAAGATT CTTAGAATTG TTTTCCATGA CGCGATCGGA 240

TTTTCGCCGG CGTTGACTGC TGCTGGTCAA TTCGGTGGTG GAGGAGCTGA TGGCTCCATC 300

ATTGCGCATT CGAACATCGA ATTGGCCTTC CCGGCTAATG GCGGCCTCAC CGACACCGTC 360

GAAGCCCTCC GCGCGGTCGG TATCAACCAC GGTGTCTCTT TCGGCGATCT CATCCAATTC 420

GCCACTGCCG TCGGCATGTC CAACTGCCCT GGCTCTCCCC GACTTGAGTT CTTGACGGGC 480

AGGAGCAACA GTTCCCAACC CTCCCCTCCT TCGTTGATCC CCGGTCCCGG AAACACTGTC 540

ACTGCTATCT TGGATCGTAT GGGCGATGCA GGCTTCAGCC CTGATGAAGT AGTTGACTTG 600

CTTGCTGCGC ATAGTTTGGC TTCTCAGGAG GGTTTGAACT CGGCCATCTT CAGGTCTCCT 660

TTGGACTCGA CCCCTCAAGT TTTCGATACC CAGTTCTACA TTGAGACCTT GCTCAAGGGT 720

ACCACTCAGC CTGGCCCTTC TCTCGGCTTT GCAGAGGAGC TCTCCCCCTT CCCTGGCGAA 780

TTCCGCATGA GGTCCGATGC TCTCTTGGCT CGCGACTCCC GAACCGCCTG CCGATGGCAA 840

TCCATGACCA GCAGCAATGA AGTTATGGGC CAGCGATACC GCGCCGCCAT GGCCAAGATG 900

TCTGTTCTCG GCTTCGACAG GAACGCCCTC ACCGATTGCT CTGACGTTAT TCCTTCTGCT 960

GTGTCCAACA ACGCTGCTCC TGTTATCCCT GGTGGCCTTA CTGTCGATGA TATCGAGGTT 1020

TCGTGCCCGA GCGAGCCTTT CCCTGAAATT GCTACCGCCT CAGGCCCTCT CCCCTCCCTC 1080

GCTCCTGCTC CTTGATCTGG TGAAGATGGT ACATCCTGCT CTCTCATCAT CCCTCTTAGC 1140

TATTTATCCA ATCTATCTAC CTATCTATGC AGTTTCTGTT CTATCACCAC AGGAAGCAAG 1200

AAAGAAAAAC AACAATGCAA CGTGAGCAGA AATCAGCAAA AAAATAAATC AGTATACTAC 1260

AGTAATGAGG CCAGTTTGCG TGGTGTCAGA AGTAAGTACG ACTCGG 1306

## Description

The present invention relates to an enzymatic composition capable of killing or inhibiting microbial cells or microorganisms, more specifically microbial cells or microorganisms present in laundry, on hard surface, on skin, teeth or mucous membranes; and for preserving food products, cosmetics, paints, coatings, etc., the composition comprising a peroxidase enzyme and an enhancing agent acting as electron donor.

### BACKGROUND OF THE INVENTION

Various enzymatic antimicrobial compositions are known in the art. For instance, WO 94/04127 discloses stabilized dentifrice compositions which are capable of producing antimicrobially effective concentrations of hypothiocyanite ions. The compositions contain an oxidoreductase capable of producing hydrogen peroxide and a peroxidase enzyme capable of oxidizing thiocyanate ions, which are normally present in saliva, to antimicrobial hypothiocyanite ions. Suitable peroxidases include lactoperoxidase, myeloperoxidase, salivary peroxidase and chloroperoxidase.

In EP-A-0 500 387 enzymatic antimicrobial compositions are disclosed comprising a haloperoxidase, e.g. myeloperoxidase, eosinophil oxidase, lactoperoxidase and chloroperoxidase, which selectively binds to and inhibits the growth of target microorganisms in the presence of peroxide and halide.

WO 95/27046 discloses an antimicrobial composition comprising a Vanadium chloroperoxidase, halide ions, and hydrogen peroxide or a hydrogen peroxide-generating agent.

The object of the invention is to provide a composition for killing or inhibiting microbial cells, i.e. for disinfection or preservation, which is easy to use and an effective alternative to the known disinfecting and preserving compositions and methods.

### SUMMARY OF THE INVENTION

Surprisingly, it has been found that the combined action of a peroxidase enzyme from the fungus *Coprinus* and an enhancing agent acting as electron-donor, when applied to e.g. a hard surface, skin, mucous membranes, oral cavity, hair, or laundry in the presence of hydrogen peroxide, results in a hitherto unknown synergistic antimicrobial effect.

Thus, based on these findings the present invention provides, in a first aspect, an enzymatic antimicrobial composition comprising or consisting essentially of a peroxidase obtainable from or produced by the fungus *Coprinus,* an enhancing agent, and hydrogen peroxide or a source of hydrogen peroxide.

The composition of the invention is useful as antimicrobial ingredient wherever such an ingredient is needed, for example for the preservation of food, beverages, cosmetics, deodorants, contact lens products, food ingredients or enzyme compositions; as a disinfectant for use e.g. on human or animal skin, hair, oral cavity, mucous membranes, wounds, bruises or in the eye; for killing microbial cells in laundry; and for incorporation in cleaning compositions or disinfectants for hard surface cleaning or disinfection.

Accordingly, in further aspects, the present invention provides a method of inhibiting microorganisms present in laundry, wherein the laundry is treated with a soaking, washing or rinsing liquor comprising this composition; a method of inhibiting microbial growth on a hard surface, wherein the surface is contacted with this composition; and a method of killing microbial cells present on human or animal skin, mucous membranes, teeth, wounds, bruises or in the eye or inhibiting the growth thereof, wherein the cells to be killed or inhibited or the skin, mucous membrane, teeth, wound or bruise are/is contacted with this composition.

### DETAILED DESCRIPTION OF THE INVENTION

The term "microbial cells" denotes bacterial or fungal cells, and the term "microorganism" denotes a fungus, a bacterium or a yeast.

The term "hard surface" as used herein relates to any surface which is essentially non-permeable for microorganisms. Examples of hard surfaces are surfaces made from metal, e.g. stainless steel, plastics, rubber, board, glass, wood, paper, textile, concrete, rock, marble, gypsum and ceramic materials which optionally may be coated, e.g. with paint, enamel and the like. The hard surface can also be a process equipment member of a cooling tower, a water treatment plant, a dairy, a food processing plant, a chemical or pharmaceutical process plant. Accordingly, the composition according to the present invention is useful in a conventional cleaning-in-place (C-I-P) system.

In the present context, the term "bactericidal" is to be understood as capable of killing bacterial cells.

In the present context, the term "bacteriostatic" is to be understood as capable of inhibiting bacterial growth, i.e. inhibiting growing bacterial cells.

In the present context, the term "fungicidal" is to be understood as capable of killing fungal cells.

In the present context, the term "fungistatic" is to be understood as capable of inhibiting fungal growth, i.e. inhibiting growing fungal cells.

Without being bound to this theory, it is believed that the key reaction in the antimicrobial effect of the combined peroxidase/enhancing agent system of the present invention is the oxidation of essential protein and enzyme sulphydryl groups.

The peroxidase enzyme is able to catalyse $H_2O_2$-dependent oxidation of an electron-donor, e.g. halide ions or the thiocyanate ion ($SCN^-$, a pseudohalide) to yield halogens or other oxidising agents. The oxidising agents make an electrophilic attack on microbial components, resulting in chemical modification of essential enzymes, transport systems, and other functional components. Sulfhydryl groups are especially susceptible to electrophilic attack, and are usually present in higher amounts than other easily oxidised groups. Aromatic amino acid residues are also susceptible to attack. Most aspects of antimicrobial action can be correlated with chemical modification of these nucleophilic components. Antimicrobial activity is favoured by influences that increase the stability of the oxidising agent, provided that these influences do not interfere with their electrophilic character, or their ability to penetrate microbial membranes. Although $H_2O_2$ itself is a powerful oxidising agent, the $H_2O_2$ molecule is stabilised and reacts slowly with biological materials. Also, most cells have enzymes that rapidly eliminate $H_2O_2$. Peroxidase-catalysed oxidation of e.g. halides or $SCN^-$ conserves the oxidising power of $H_2O_2$ in forms that react more rapidly, and for which the target cells may have no defense. (Thomas, E.L. in "The Lactoperoxidase System". Ed. By Pruitt, K.M., and Tenovuo, J.O., New York, 1985).

The reaction catalysed by peroxidase can be written as

$$H_2O_2 + AH_2 \rightarrow 2\,H_2O + A$$

where $AH_2$ and A are reduced and oxidised forms of suitable electron donors; or, in case of halides or thiocyanate,

$$H_2O_2 + X^- \rightarrow H_2O + OX^-$$

The hydrogen peroxide may be generated by an oxidoreductase enzyme and a substrate specific to that enzyme, in particular by those oxidoreductases which utilise water as a co-reactant and oxygen as an electron donor. Suitable oxidoreductases include glucose oxidase, galactose oxidase, glycolate oxidase, lactate oxidase, L-gulunolactone oxidase, L-2-hydroxyacid oxidase, aldehyde oxidase, xanthine oxidase, D-aspartate oxidase, L-amino acid oxidase, D-amino acid oxidase, monoamine oxidase, pyridoxaminephosphate oxidase, diamine oxidase, and sulfite oxidase. Glucose oxidase is most preferred. Suitable substrates are specific to the particular oxidoreductases chosen and are well known to the skilled person. For example, beta-D-glucose is a specific substrate for glucose oxidase. Other suitable substrates include, but are not limited to D-glucose, D-galactose, L-sorbose, ethanol, tyramine, 1,4-diaminobutane, 2-aminophenol, glycolate, L-lactate, 2-deoxy-D-glucose, L-gulunolactone, L-galaconolactone, D-mannonolactone, L-2-hydroxyisocaproate, acetaldehyde, butyraldehyde, xanthine, D-aspatate, D-glutamate, L-amino acids and D-amino acids.

It may be advantageous to use enzymatically generated hydrogen peroxide, since this source results in a relatively low concentration of hydrogen peroxide under the biologically relevant conditions. Low concentrations of hydrogen peroxide result in an increase in the rate of peroxidase-catalysed reaction.

The hydrogen peroxide can also be added to the composition *per se* or can be generated by perborate or percarbonate salts, preferably sodium percarbonate or sodium perborate.

**The enzyme**

The peroxidase employed in the method of the invention is producible by fungi including strains belonging to the subdivision Basidiomycotina, class Basidiomycetes, especially the genus *Coprinus*, in particular *Coprinus cinereus* f. *microsporus* (IFO 8371), or *Coprinus macrorhizus*.

The peroxidase enzyme to be used in the method of the invention may be a monocomponent (recombinant) enzyme, i.e. enzymes essentially free from other proteins or enzyme proteins. A recombinant enzyme may be cloned and expressed according to standard techniques conventional to the skilled person. However, the enzyme may also be used in the form of an enzyme preparation optionally enriched in an enzyme exhibiting the desired enzyme activity as the major enzymatic component, e.g. a mono-component enzyme preparation.

Particularly, a recombinantly produced peroxidase is a peroxidase derived from a *Coprinus* sp., in particular C. *macrorhizus* or *C. cinereus* according to WO 92/16634, or a variant thereof, e.g., a variant as described in WO 94/12621. Accordingly, a useful recombinant peroxidase may be produced by using a DNA construct comprising the

DNA sequence shown in SEQ ID No. 1 encoding a *Coprinus* sp. peroxidase, or a suitable modification thereof.

Examples of suitable modifications of the DNA sequence are nucleotide substitutions which do not give rise to another amino acid sequence of the peroxidase, but which correspond to the codon usage of the host organism, into which the DNA construct is introduced or nucleotide substitutions which do give rise to a different amino acid sequence and therefore, possibly, a different protein structure which might give rise to a peroxidase mutant with different properties than the native enzyme. Other examples of possible modifications are insertion of one or more nucleotides into the sequence, addition of one or more nucleotides at either end of the sequence, or deletion of one or more nucleotides at either end or within the sequence.

The DNA construct encoding the peroxidase may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, annealed, ligated and cloned in suitable vectors.

The DNA construct may also be of genomic or cDNA origin, for instance obtained by preparing a genomic or cDNA library and screening for DNA sequences coding for all or part of the peroxidase by hybridization using synthetic oligonucleotide probes in accordance with standard techniques (cf. Sambrook et al., Molecular Cloning: A Laboratory Manual, 2nd Ed., Cold Spring Harbor, 1989). In this case, a genomic or cDNA sequence encoding the peroxidase may be modified at a site corresponding to the site(s) at which it is desired to introduce amino acid substitutions, e.g. by site-directed mutagenesis using synthetic oligonucleotides encoding the desired amino acid sequence for homologous recombination in accordance with well-known procedures.

Finally, the DNA construct may be of mixed synthetic and genomic, mixed synthetic and cDNA or mixed genomic and cDNA origin prepared by ligating fragments of synthetic, genomic or cDNA origin (as appropriate), the fragments corresponding to various parts of the entire DNA construct, in accordance with standard techniques. The DNA construct may also be prepared by polymerase chain reaction using specific primers, for instance as described in US 4,683,202 or R.K. Saiki et al., Science 239, 1988, pp. 487-491.

The DNA construct is normally inserted into a recombinant expression vector. This may be any vector which may conveniently be subjected to recombinant DNA procedures, and the choice of vector will often depend on the host cell, into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s), into which it has been integrated.

In the vector, the DNA sequence encoding the peroxidase should be operably connected to a suitable promoter and terminator sequence. The promoter may be any DNA sequence which shows transcriptional activity in the host cell of choice and may be derived from genes encoding proteins either homologous or heterologous to the host cell.

Examples of suitable promoters are those indicated above. The procedures used to ligate the DNA sequences coding for the peroxidase, the promoter and the terminator, respectively, and to insert them into suitable vectors are well known to persons skilled in the art (cf., for instance, Sambrook et al., op.cit.).

A host cell is transformed with the expression vector. The host cell is a cell of a filamentous fungus, and is preferably a cell of an *Aspergillus sp.* as indicated above.

The medium used to culture the transformed host cells may be any conventional medium suitable for growing filamentous fungi. The transformants are usually stable and may be cultured in the absence of selection pressure. However, if the transformants are found to be unstable, a selection marker introduced into the cells may be used for selection. If hemin or a heme-containing material (e.g. hemoglobin or red blood cells) is added to the medium, the yield of heme protein may be significantly increased.

The mature heme protein secreted from the host cells may conveniently be recovered from the culture medium by well-known procedures including separating the cells from the medium by centrifugation or filtration, and precipitating proteinaceous components of the medium by means of a salt such as ammonium sulphate, followed by chromatographic procedures such as ion exchange chromatography, affinity chromatography, or the like.

In the context of this invention, peroxidase acting compounds comprise peroxidase active fragments derived from cytochromes, haemoglobin or peroxidase enzymes, and synthetic or semisynthetic derivatives thereof, e.g. iron porphins, iron porphyrins, and iron phthalocyanine and derivatives thereof.

Determination of peroxidase activity: 1 peroxidase unit (POXU) is the amount of enzyme that catalyzes the conversion of 1 µmol hydrogen peroxide per minute at the following analytical conditions: 0.88 mM hydrogen peroxide, 1.67 mM 2,2'-azinobis(3-ethylbenzothiazoline-6-sulfonate), 0.1 M phosphate buffer, pH 7.0, incubated at 30°C, photometrically followed at 418 nm; molecular extinction coefficient $\varepsilon = 3.6 \times 10^4\ M^{-1} \cdot cm^{-1}$.

The peroxidase enzyme may be present in the composition of the invention corresponding to 0.01-100 POXU per ml of ready-to-use liquid, i.e. of washing solution, disinfecting liquid, preserving liquid, foot bath etc.

**The enhancing agents**

In a preferred embodiment of the invention, the enhancing agent capable of acting as an electron-donor is a source of ionic iodide which may be enzymatically converted to iodine when contacted with peroxidase enzyme in an aqueous solution for a time and under conditions sufficient to permit the conversion.

Iodine ($I_2$) is widely used as a disinfectant, for many types of situations, for example as skin cleansers, for wound disinfection, contact lens cleaning and water sanitation, to mention a few. In addition, iodine is also useful in catalysts, as an animal feed additive, in pharmaceuticals, and as polymer precursor additives. Although the $I_2$-based system of disinfection is extremely effective, several factors limit the scope of directly applying $I_2$. In particular, the storage, transportation and handling of $I_2$ are extremely hazardous, due to the chemicals involved in production and also due to the toxicity of $I_2$ itself even in moderate concentrations. Generally, $I_2$ is obtained from natural sources, such as brine, by processes that utilise strong inorganic acids, chlorine gas, and other hazardous chemicals. Iodophores have been developed as $I_2$ carriers to replace simple $I_2$ solutions for industrial and domestic disinfection. In addition, binary systems capable of generating $I_2$ from an $I^-$ salt and a chemical oxidant are also available. Both these systems create the need for disposal of large, potentially toxic amounts of by-products. Another alternative to both industrially producing $I_2$ on a large scale, and to applying $I_2$ as a disinfectant, has been found in the peroxidase-based generation of $I_2$ (US 4,282,324; US 4,617,190; US 4,588,586; US 4,937,072; US 5,055,287; US 5,227,161; US 5,169,455; US 4,996,146; US 4,576,817). Such methods involve the use of a peroxidase enzyme, the oxidising agent $H_2O_2$, and a source of ionic iodide, Unfortunately, this method has the disadvantage of requiring the hazardous and volatile peroxide or peracid, which has to be either transported or generated *in situ* by additional enzymatic or chemical steps, this making the system more complex and/or costly.

In the present context, a preferred source of ionic iodide is a water-soluble iodide salt such as an alkaline metal iodide salt, e.g. potassium iodide (KI), sodium iodide (NaI), or lithium iodide, ammonium iodide, calcium iodide. Sodium iodide and potassium iodide are preferred.

Another preferred enhancing agent is a source of the thiocyanate ion ($SCN^-$), e.g. sodium thiocyanate, potassium thiocyanate, ammonium thiocyanate, and other thiocyanate salts, preferably sodium thiocyanate and potassium thiocyanate.

In another preferred embodiment, a useful enhancing agent is the compound described by the following formula:

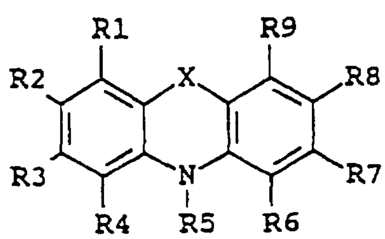

in which formula X represents (-O-) or (-S-), and the substituent groups $R^1$-$R^9$, which may be identical or different, independently represents any of the following radicals: hydrogen, halogen, hydroxy, formyl, carboxy, and esters and salts hereof, carbamoyl, sulfo, and esters and salts hereof, sulfamoyl, nitro, amino, phenyl, $C_1$-$C_{14}$-alkyl, $C_1$-$C_5$-alkoxy, carbonyl-$C_1$-$C_5$-alkyl, aryl-$C_1$-$C_5$-alkyl; which carbamoyl, sulfamoyl, and amino groups may furthermore be unsubstituted or substituted once or twice with a substituent group $R^{10}$; and which phenyl may furthermore be unsubstituted or substituted with one or more substituent groups $R^{10}$; and which $C_1$-$C_{14}$-alkyl, $C_1$-$C_5$-alkoxy, carbonyl-$C_1$-$C_5$-alkyl, and aryl-$C_1$-$C_5$-alkyl groups may be saturated or unsaturated, branched or unbranched, and may furthermore be unsubstituted or substituted with one or more substituent groups $R^{10}$;
which substituent group $R^{10}$ represents any of the following radicals: halogen, hydroxy, formyl, carboxy and esters and salts hereof, carbamoyl, sulfo and esters and salts hereof, sulfamoyl, nitro, amino, phenyl, aminoalkyl, piperidino, piperazinyl, pyrrolidin-1-yl, $C_1$-$C_5$-alkyl, $C_1$-$C_5$-alkoxy; which carbamoyl, sulfamoyl, and amino groups may furthermore be unsubstituted or substituted once or twice with hydroxy, $C_1$-$C_5$-alkyl, $C_1$-$C_5$-alkoxy; and which phenyl may furthermore be substituted with one or more of the following radicals: halogen, hydroxy, amino, formyl, carboxy and esters and salts hereof, carbamoyl, sulfo and esters and salts hereof, and sulfamoyl; and which $C_1$-$C_5$-alkyl, and $C_1$-$C_5$-alkoxy groups may furthermore be saturated or unsaturated, branched or unbranched, and may furthermore be substituted once or twice with any of the following radicals: halogen, hydroxy, amino, formyl, carboxy and esters and salts hereof,

carbamoyl, sulfo and esters and salts hereof, and sulfamoyl;
or in which general formula two of the substituent groups $R^1$-$R^9$ may together form a group -B-, in which B represents any of the following the groups: (-$CHR^{10}$-N=N-), $(-CH=CH-)_n$, $(-CH=N-)_n$ or (-N=$CR^{10}$-$NR^{11}$-), in which groups n-represents an integer of from 1 to 3, $R^{10}$ is a substituent group as defined above and $R^{11}$ is defined as $R^{10}$. (It is to be understood that if the above mentioned formula comprises two or more $R^{10}$-substituent groups, these $R^{10}$-substituent groups may be the same or different).

In particular embodiments, the enhancing agent is 10-methylphenothiazine, phenothiazine-10-propionic acid, N-hydroxysuccinimide phenothiazine-10-propionate, 10-ethylphenothiazine-4-carboxylic acid, 10-ethylphenothiazine, 10-propylphenothiazine, 10-isopropylphenothiazine, methyl phenothiazine-10-propionate, 10-phenylphenothiazine, 10-allylphenothiazine, 10-(3-(4-methylpiperazin-1-yl)propyl)phenothiazine, 10-(2-pyrrolidin-1-yl-ethyl)phenothiazine, 2-methoxy-10-methyl-phenothiazine, 1-methoxy-10-methylphenothiazine, 3-methoxy-10-methylphenothiazine, 3,10-dimethylphenothiazine, 3,7,10-trimethylphenothiazine, 10-(2-hydroxyethyl)phenothiazine, 10-(3-hydroxypropyl) phenothiazine, 3-(2-hydroxyethyl)-10-methylphenothiazine, 3-hydroxymethyl-10-methylphenothiazine, 3,7-dibromophenothiazine-10-propionic acid, phenothiazine-10-propionamide, chlorpromazine, 2-chloro-10-methylphenothiazine, 2-acetyl-10-methylphenothiazine, 10-methylphenoxazine, 10-ethylphenoxazine, phenoxazine-10-propionic acid, 10-(2-hydroxyethyl)phenoxazine or 4-carboxyphenoxazine-10-propionic acid.

Another example of a useful enhancing agent is a compound described by the following formula:

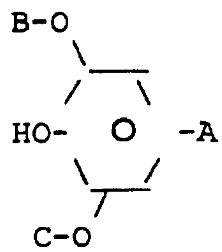

in which formula A is a group such as -D, -CH=CH-D, -CH=CH-CH=CH-D, -CH=N-D, -N=N-D, or -N=CH-D, in which D is selected from the group consisting of -CO-E, -$SO_2$-E, -N-XY, and -$N^+$-XYZ, in which E may be -H, -OH, -R, or -OR, and X and Y and Z may be identical or different and selected from -H and -R; R being a $C_1$-$C_{16}$ alkyl, preferably a $C_1$-$C_8$ alkyl, which alkyl may be saturated or unsaturated, branched or unbranched and optionally substituted with a carboxy, sulfo or amino group; and B and C may be the same or different and selected from $C_mH_{2m+1}$; $1 \le m \le 5$.

In a preferred embodiment A in the above mentioned formula is -CO-E, in which E may be -H, -OH, -R, or -OR; R being a $C_1$-$C_{16}$ alkyl, preferably a $C_1$-$C_8$ alkyl, which alkyl may be saturated or unsaturated, branched or unbranched and optionally substituted with a carboxy, sulfo or amino group; and B and C may be the same or different and selected from $C_mH_{2m+1}$; $1 \le m \le 5$.

In the above mentioned formula A may be placed meta to the hydroxy group instead of being placed in the paraposition as shown.

In particular embodiments, the enhancing agent is acetosyringone, methylsyringate, ethylsyringate, propylsyringate, butylsyringate, hexylsyringate, or octylsyringate.

Yet another useful enhancing agent is an azino compound described by the general formula

$$A=N-N=B$$

in which formula the symbols A and B, which may be identical or different, independently represent any of the substituents II, III, IV, and V,

(II)

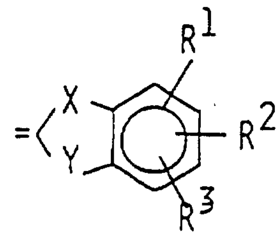

(III)

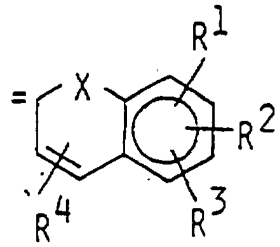

(IV)

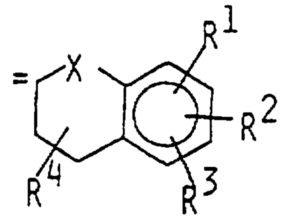

(V)

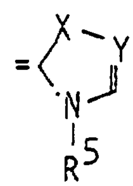

in which substituents the symbols X and Y, which may be identical or different, independently represent carbon, nitrogen, which nitrogen may be unsubstituted or substituted with a substituent group $R^5$, sulfur, oxygen, selenium or tellurium; and in which substituents the substituent groups $R^1$, $R^2$, $R^3$, and $R^4$, which may be identical or different, independently represent hydrogen, halogen, a hydroxy group, a $C_1$-$C_3$ alkoxy group, a formyl group, a carboxy group, a sulfo group, a nitro group, a $C_1$-$C_5$ alkyl group, which alkyl group may furthermore be saturated or unsaturated, linear or branched, or an amino group, which amino group may furthermore be unsubstituted or substituted once or twice with a substituent group $R^5$;

which substituent group $R^5$ represents halogen, a hydroxy group, a $C_1$-$C_3$ alkoxy group, a $C_1$-$C_5$ alkyl group, or an amino group.

The peroxidase enhancing agent may be in free form or in the form of an addition salt.

In preferred embodiments, the substituent groups $R^1$, $R^2$, $R^3$, and $R^4$, which may be identical or different, independently represent hydrogen, halogen, a hydroxy group, a $C_1$-$C_3$ alkyl group, or a sulfo group. Preferably, the halogen is fluoro, chloro, or bromo. Preferably, the $C_1$-$C_3$ alkyl group is methyl, ethyl, propyl, or isopropyl.

In preferred embodiments, the substituent group $R^5$ represents halogen, a hydroxy group, a $C_1$-$C_3$ alkoxy group, a $C_1$-$C_3$ alkyl group, or an amino group.

In a most preferred embodiment, a peroxidase enhancing agent of the invention is 2,2'-azino-bis(3-ethylbenzothiazoline-6-sulfonate). This compound, abbreviated ABTS, is a chromogenic substrate, and a common peroxidase and phenol oxidase assay agent.

It has, moreover, been demonstrated that ABTS, contrary to the enhancers known and described above, is

capable of acting as a peroxidase enhancing agent at highly alkaline conditions, i.e. above pH 9. This feature allows ABTS to be implemented into e.g. detergent compositions, intended for performance in the range pH 7-13, particularly the range pH 8-12, preferably the range pH 9-11.

The enhancing agent may be present in the antimicrobial composition in concentrations corresponding to from 0.005 to 1000 µmole per g of substrate (microbial cells, biomass), preferably 0.05 to 500 µmole per g of substrate, more preferably 0.5 to 100 µmole per g of substrate.

**Stability of the Radical of the Enhancing Agent**

Without being limited to any theory it is presently contemplated that there is a positive correlation between the half-life of the radical which the enhancing agent forms in the relevant aqueous medium and its efficiency, and that this half-life is significantly longer than the half-life of any of the substances selected from the group consisting of p-hydroxycinnamic acid, 2,4-dichlorophenol, p-hydroxybenzene sulphonate, vanillin and p-hydroxybenzoic acid (i.e. the enhancing agents disclosed in WO 92/18683).

As the half-life of the radical is dependent on, inter alia, the pH, the temperature and the buffer of the aqueous medium, it is very important that all these factors are the same when the half-lives of the radicals of various enhancing agents are compared.

**The composition**

The enzymatic composition of the invention may further comprise auxiliary agents such as wetting agents, thickening agents, buffer, stabilisers, perfume, colourants, fillers and the like.

Useful wetting agents are surfactants, i.e. non-ionic, anionic, amphoteric or zwitterionic surfactants. Examples of useful surfactants are mentioned below under "Uses";

The composition of the invention may be used in the form of a powder which is to be dissolved in water prior to use, or may be a gelled product or a liquid product. The composition may be a concentrated product or a ready-to-use product.

In use, the concentrated product is typically diluted with water to provide a medium having an effective antimicrobial activity, applied to the object to be disinfected or preserved, and allowed to react with the microorganisms present.

The optimum pH condition is usually a compromise between optimum stability and optimum activity of the peroxidase enzyme, optimum stability and optimum reactivity (oxidation potential) of the radical of the enhancing agent, and the choice of buffering system.

**Uses**

The composition of the invention may be incorporated into a detergent or cleaning composition comprising more enzyme types useful in detergent or cleaning compositions, preferably at least one further enzyme selected from the group consisting of proteases, amylases, cutinases, peroxidases, oxidases , laccases, cellulases, xylanases, and lipases.

Surfactant system

The detergent compositions according to the present invention comprise a surfactant system, wherein the surfactant can be selected from nonionic and/or anionic and/or cationic and/or ampholytic and/or zwitterionic and/or semi-polar surfactants.

The surfactant is typically present at a level from 0.1% to 60% by weight.

The surfactant is preferably formulated to be compatible with enzyme components present in the composition. In liquid or gel compositions the surfactant is most preferably formulated in such a way that it promotes, or at least does not degrade, the stability of any enzyme in these compositions.

Preferred systems to be used according to the present inven-tion comprise as a surfactant one or more of the nonionic and/or anionic surfactants described herein.

Polyethylene, polypropylene, and polybutylene oxide condensates of alkyl phenols are suitable for use as the nonionic surfactant of the surfactant systems of the present invention, with the polyethylene oxide condensates being preferred. These compounds include the condensation products of alkyl phenols having an alkyl group containing from about 6 to about 14 carbon atoms, preferably from about 8 to about 14 carbon atoms, in either a straight chain or branched-chain configuration with the alkylene oxide. In a preferred embodiment, the ethylene oxide is present in an amount equal to from about 2 to about 25 moles, more preferably from about 3 to about 15 moles, of ethylene oxide

per mole of alkyl phenol. Commercially available nonionic surfactants of this type include Igepal™ CO-630, marketed by the GAF Corporation; and Triton™ X-45, X-114, X-100 and X-102, all marketed by the Rohm & Haas Company. These surfactants are commonly referred to as alkylphenol alkoxylates (e.g., alkyl phenol ethoxylates).

The condensation products of primary and secondary aliphatic alcohols with about 1 to about 25 moles of ethylene oxide are suitable for use as the nonionic surfactant of the nonionic surfactant systems of the present invention. The alkyl chain of the aliphatic alcohol can either be straight or branched, primary or secondary, and generally contains from about 8 to about 22 carbon atoms. Preferred are the condensation products of alcohols having an alkyl group containing from about 8 to about 20 carbon atoms, more preferably from about 10 to about 18 carbon atoms, with from about 2 to about 10 moles of ethylene oxide per mole of alcohol. About 2 to about 7 moles of ethylene oxide and most preferably from 2 to 5 moles of ethylene oxide per mole of alcohol are present in said condensation products. Examples of commercially available nonionic surfactants of this type include Tergitol™ 15-S-9 (The condensation product of $C_{11}$-$C_{15}$ linear alcohol with 9 moles ethylene oxide), Tergitol™ 24-L-6 NMW (the condensation product of $C_{12}$-$C_{14}$ primary alcohol with 6 moles ethylene oxide with a narrow molecular weight distribution), both marketed by Union Carbide Corporation; Neodol™ 45-9 (the condensation product of $C_{14}$-$C_{15}$ linear alcohol with 9 moles of ethylene oxide), Neodol™ 23-3 (the condensation product of $C_{12}$-$C_{13}$ linear alcohol with 3.0 moles of ethylene oxide), Neodol™ 45-7 (the condensation product of $C_{14}$-$C_{15}$ linear alcohol with 7 moles of ethylene oxide), Neodol™ 45-5 (the condensation product of $C_{14}$-$C_{15}$ linear alcohol with 5 moles of ethylene oxide) marketed by Shell Chemical Company, Kyro™ EOB (the condensation product of $C_{13}$-$C_{15}$ alcohol with 9 moles ethylene oxide), marketed by The Procter & Gamble Company, and Genapol LA 050 (the condensation product of $C_{12}$-$C_{14}$ alcohol with 5 moles of ethylene oxide) marketed by Hoechst. Preferred range of HLB in these products is from 8-11 and most preferred from 8-10.

Also useful as the nonionic surfactant of the surfactant systems of the present invention are alkylpolysaccharides disclosed in US 4,565,647, having a hydrophobic group containing from about 6 to about 30 carbon atoms, preferably from about 10 to about 16 carbon atoms and a polysaccharide, e.g. a polyglycoside, hydrophilic group containing from about 1.3 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties (optionally the hydrophobic group is attached at the 2-, 3-, 4-, etc. positions thus giving a glucose or galactose as opposed to a glucoside or galactoside). The intersaccharide bonds can be, e.g., between the one position of the additional saccharide units and the 2-, 3-, 4-, and/or 6- positions on the preceding saccharide units.

The preferred alkylpolyglycosides have the formula

$$R^2O(C_nH_{2n}O)_t(glycosyl)_x$$

wherein $R^2$ is selected from the group consisting of alkyl, alkylphenyl, hydroxyalkyl, hydroxyalkylphenyl, and mixtures thereof in which the alkyl groups contain from about 10 to about 18, preferably from about 12 to about 14, carbon atoms; n is 2 or 3, preferably 2; t is from 0 to about 10, pre-ferably 0; and x is from about 1.3 to about 10, preferably from about 1.3 to about 3, most preferably from about 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4-, and/or 6-position, preferably predominantly the 2-position.

The condensation products of ethylene oxide with a hydrophobic base formed by the condensation of propylene oxide with propylene glycol are also suitable for use as the additional nonionic surfactant systems of the present invention. The hydrophobic portion of these compounds will preferably have a molecular weight from about 1500 to about 1800 and will exhibit water insolubility. The addition of polyoxyethylene moieties to this hydrophobic portion tends to increase the water solubility of the molecule as a whole, and the liquid character of the product is retained up to the point where the polyoxyethylene content is about 50% of the total weight of the condensation product, which corresponds to condensation with up to about 40 moles of ethylene oxide. Examples of compounds of this type include certain of the commercially available Pluronic™ surfactants, marketed by BASF.

Also suitable for use as the nonionic surfactant of the nonionic surfactant system of the present invention, are the condensation products of ethylene oxide with the product resulting from the reaction of propylene oxide and ethylenediamine. The hydrophobic moiety of these products consists of the reaction product of ethylenediamine and excess propylene oxide, and generally has a molecular weight of from about 2500 to about 3000. This hydrophobic moiety is condensed with ethylene oxide to the extent that the condensation product contains from about 40% to about 80% by weight of polyoxyethylene and has a molecular weight of from about 5,000 to about 11,000. Examples of this type of nonionic surfactant include certain of the commercially available Tetronic™ compounds, marketed by BASF.

Preferred for use as the nonionic surfactant of the surfactant systems of the present invention are polyethylene

oxide condensates of alkyl phenols, condensation products of primary and secondary aliphatic alcohols with from about 1 to about 25 moles of ethyleneoxide, alkylpolysaccharides, and mixtures hereof. Most preferred are $C_8$-$C_{14}$ alkyl phenol ethoxylates having from 3 to 15 ethoxy groups and $C_8$-$C_{18}$ alcohol ethoxylates (preferably $C_{10}$ avg.) having from 2 to 10 ethoxy groups, and mixtures thereof.

Highly preferred nonionic surfactants are polyhydroxy fatty acid amide surfactants of the formula

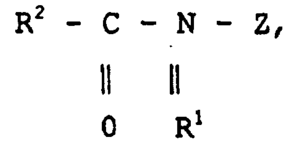

wherein $R^1$ is H, or $R^1$ is $C_{1-4}$ hydrocarbyl, 2-hydroxyethyl, 2-hydroxypropyl or a mixture thereof, $R^2$ is $C_{5-31}$ hydrocarbyl, and Z is a polyhydroxyhydrocarbyl having a linear hydrocarbyl chain with at least 3 hydroxyls directly connected to the chain, or an alkoxylated derivative thereof. Preferably, $R^1$ is methyl, $R^2$ is straight $C_{11-15}$ alkyl or $C_{16-18}$ alkyl or alkenyl chain such as coconut alkyl or mixtures thereof, and Z is derived from a reducing sugar such as glucose, fructose, maltose or lactose, in a reductive amination reaction.

Highly preferred anionic surfactants include alkyl alkoxylated sulfate surfactants. Examples hereof are water soluble salts or acids of the formula $RO(A)_mSO_3M$ wherein R is an unsubstituted $C_{10}$-C-$_{24}$ alkyl or hydroxyalkyl group having a $C_{10}$-$C_{24}$ alkyl component, preferably a $C_{12}$-$C_{20}$ alkyl or hydro-xyalkyl, more preferably $C_{12}$-$C_{18}$ alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethyl-ammonium cations and quaternary ammonium cations such as tetramethyl-ammonium and dimethyl piperdinium cations and those derived from alkylamines such as ethylamine, diethylamine, triethylamine, mixtures thereof, and the like. Exemplary surfactants are $C_{12}$-$C_{18}$ alkyl polyethoxylate (1.0) sulfate ($C_{12}$-$C_{18}$E(1.0)M), $C_{12}$-$C_{18}$ alkyl polyethoxylate (2.25) sulfate ($C_{12}$-$C_{18}$(2.25)M, and $C_{12}$-$C_{18}$ alkyl polyethoxylate (3.0) sulfate ($C_{12}$-$C_{18}$E(3.0)M), and $C_{12}$-$C_{18}$ alkyl polyethoxylate (4.0) sulfate ($C_{12}$-$C_{18}$E(4.0)M), wherein M is conveniently selected from sodium and potassium. Suitable anionic surfactants to be used are alkyl ester sulfonate surfactants including linear esters of $C_8$-$C_{20}$ carboxylic acids (i.e., fatty acids) which are sulfonated with gaseous $SO_3$ according to "The Journal of the American Oil Chemists Society", 52 (1975), pp. 323-329. Suitable starting materials would include natural fatty substances as derived from tallow, palm oil, etc.

The preferred alkyl ester sulfonate surfactant, especially for laundry applications, comprise alkyl ester sulfonate surfactants of the structural formula:

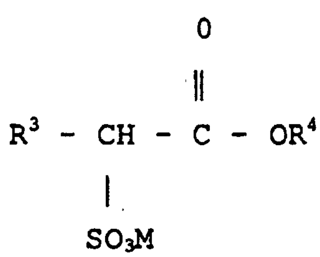

wherein $R^3$ is a $C_8$-$C_{20}$ hydrocarbyl, preferably an alkyl, or combination thereof, $R^4$ is a $C_1$-$C_6$ hydrocarbyl, preferably an alkyl, or combination thereof, and M is a cation which forms a water soluble salt with the alkyl ester sulfonate. Suitable salt-forming cations include metals such as sodium, potassium, and lithium, and substituted or unsubstituted ammonium cations, such as monoethanolamine, diethonolamine, and triethanolamine. Preferably, $R^3$ is $C_{10}$-$C_{16}$ alkyl, and $R^4$ is methyl, ethyl or isopropyl. Especially preferred are the methyl ester sulfonates wherein $R^3$ is $C_{10}$-$C_{16}$ alkyl.

Other suitable anionic surfactants include the alkyl sulfate surfactants which are water soluble salts or acids of the formula $ROSO_3M$ wherein R preferably is a $C_{10}$-$C_{24}$ hydrocarbyl, preferably an alkyl or hydroxyalkyl having a $C_{10}$-$C_{20}$ alkyl component, more preferably a $C_{12}$-$C_{18}$ alkyl or hydroxyalkyl, and M is H or a cation, e.g., an alkali metal cation (e.g. sodium, potassium, lithium), or ammonium or substituted ammonium (e.g. methyl-, dimethyl-, and trimethyl ammonium cations and quaternary ammonium cations such as tetramethyl-ammonium and dimethyl piperdinium cations and quaternary ammonium cations derived from alkylamines such as ethylamine, diethylamine, triethylamine, and mixtures thereof, and the like). Typically, alkyl chains of $C_{12}$-$C_{16}$ are preferred for lower wash temperatures (e.g. below about 50°C) and $C_{16}$-$C_{18}$ alkyl chains are preferred for higher wash temperatures (e.g. above about 50°C).

Other anionic surfactants useful for detersive purposes can also be included in the laundry detergent compositions of the present invention. Theses can include salts (including, for example, sodium, potassium, ammonium, and substituted ammonium salts such as mono- di- and triethanolamine salts) of soap, $C_8$-$C_{22}$ primary or secondary alkanesulfonates, $C_8$-$C_{24}$ olefinsulfonates, sulfonated polycarboxylic acids prepared by sulfonation of the pyrolyzed product of alkaline earth metal citrates, e.g., as described in British patent specification No. 1,082,179, $C_8$-$C_{24}$ alkylpolyglycolethersulfates (containing up to 10 moles of ethylene oxide); alkyl glycerol sulfonates, fatty acyl glycerol sulfonates, fatty oleyl glycerol sulfates, alkyl phenol ethylene oxide ether sulfates, paraffin sulfonates, alkyl phosphates, isethionates such as the acyl isethionates, N-acyl taurates, alkyl succinamates and sulfosuccinates, monoesters of sulfosuccinates (especially saturated and unsaturated $C_{12}$-$C_{18}$ monoesters) and diesters of sulfosuccinates (especially saturated and unsaturated $C_6$-$C_{12}$ diesters), acyl sarcosinates, sulfates of alkylpolysaccharides such as the sulfates of alkylpolyglucoside (the nonionic nonsulfated compounds being described below), branched primary alkyl sulfates, and alkyl polyethoxy carboxylates such as those of the formula $RO(CH_2CH_2O)_k$-$CH_2COO$-M+ wherein R is a $C_8$-$C_{22}$ alkyl, k is an integer from 1 to 10, and M is a soluble salt forming cation. Resin acids and hydrogenated resin acids are also suitable, such as rosin, hydrogenated rosin, and resin acids and hydrogenated resin acids present in or derived from tall oil.

Alkylbenzene sulfonates are highly preferred. Especially preferred are linear (straight-chain) alkyl benzene sulfonates (LAS) wherein the alkyl group preferably contains from 10 to 18 carbon atoms.

Further examples are described in "Surface Active Agents and Detergents" (Vol. I and II by Schwartz, Perrry and Berch). A variety of such surfactants are also generally disclosed in US 3,929,678, (Column 23, line 58 through Column 29, line 23, herein incorporated by reference).

When included therein, the laundry detergent compositions of the present invention typically comprise from about 1% to about 40%, preferably from about 3% to about 20% by weight of such anionic surfactants.

The laundry detergent compositions of the present invention may also contain cationic, ampholytic, zwitterionic, and semi-polar surfactants, as well as the nonionic and/or anionic surfactants other than those already described herein.

Cationic detersive surfactants suitable for use in the laundry detergent compositions of the present invention are those having one long-chain hydrocarbyl group. Examples of such cationic surfactants include the ammonium surfactants such as alkyltrimethylammonium halogenides, and those surfactants having the formula:

$$[R^2 (OR^3)_y] [R^4 (OR^3)_y]_2 R^5 + X-$$

wherein $R^2$ is an alkyl or alkyl benzyl group having from about 8 to about 18 carbon atoms in the alkyl chain, each $R^3$ is selected form the group consisting of $-CH_2CH_2-$, $- CH_2CH(CH_3)-$, $-CH_2CH(CH_2OH)-$, $-CH_2CH_2CH_2-$, and mixtures thereof; each $R^4$ is selected from the group consisting of $C_1$-$C_4$ alkyl, $C_1$-$C_4$ hydroxyalkyl, benzyl ring structures formed by joining the two $R^4$ groups, $-CH_2CHOHCHOHCOR^6CHOHCH_2OH$, wherein $R^6$ is any hexose or hexose polymer having a molecular weight less than about 1000, and hydrogen when y is not 0; $R^5$ is the same as $R^4$ or is an alkyl chain,wherein the total number of carbon atoms or $R^2$ plus $R^5$ is not more than about 18; each y is from 0 to about 10, and the sum of the y values is from 0 to about 15; and X is any compatible anion.

Highly preferred cationic surfactants are the water soluble quaternary ammonium compounds useful in the present composition having the formula:

$$R_1R_2R_3R_4N^+X^- \qquad (i)$$

wherein $R_1$ is $C_8$-$C_{16}$ alkyl, each of $R_2$, $R_3$ and $R_4$ is independently $C_1$-$C_4$ alkyl, $C_1$-$C_4$ hydroxy alkyl, benzyl, and $-(C_2H_4)_xH$ where x has a value from 2 to 5, and X is an anion. Not more than one of $R_2$, $R_3$ or $R_4$ should be benzyl.

The preferred alkyl chain length for $R_1$ is $C_{12}$-$C_{15}$, particularly where the alkyl group is a mixture of chain lengths derived from coconut or palm kernel fat or is derived synthetically by olefin build up or OXO alcohols synthesis.

Preferred groups for $R_2R_3$ and $R_4$ are methyl and hydroxyethyl groups and the anion X may be selected from halide, methosulphate, acetate and phosphate ions. Examples of suitable quaternary ammonium compounds of formulae (i) for use herein are:

coconut trimethyl ammonium chloride or bromide;

coconut methyl dihydroxyethyl ammonium chloride or bromide;
decyl triethyl ammonium chloride;
decyl dimethyl hydroxyethyl ammonium chloride or bromide; $C_{12-15}$ dimethyl hydroxyethyl ammonium chloride or bromide;
coconut dimethyl hydroxyethyl ammonium chloride or bromide;
myristyl trimethyl ammonium methyl sulphate;
lauryl dimethyl benzyl ammonium chloride or bromide;
lauryl dimethyl (ethenoxy)$_4$ ammonium chloride or bromide;
choline esters (compounds of formula (i) wherein $R_1$ is

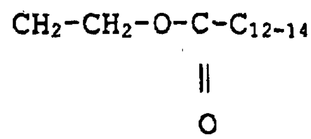

alkyl and $R_2R_3R_4$ are methyl).

di-alkyl imidazolines [compounds of formula (i)].
Other cationic surfactants useful herein are also described in US 4,228,044 and in EP 000 224.
When included therein, the laundry detergent compositions of the present invention typically comprise from 0.2% to about 25%, preferably from about 1% to about 8% by weight of such cationic surfactants.
Ampholytic surfactants are also suitable for use in the laundry detergent compositions of the present invention. These surfactants can be broadly described as aliphatic derivatives of secondary or tertiary amines, or aliphatic derivatives of heterocyclic secondary and tertiary amines in which the aliphatic radical can be straight- or branched-chain. One of the aliphatic substituents contains at least about 8 carbon atoms, typically from about 8 to about 18 carbon atoms, and at least one contains an anionic water-solubilizing group, e.g. carboxy, sulfonate, sulfate.
See US 3,929,678 (column 19, lines 18-35) for examples of ampholytic surfactants.
When included therein, the laundry detergent compositions of the present invention typically comprise from 0.2% to about 15%, preferably from about 1% to about 10% by weight of such ampholytic surfactants.
Zwitterionic surfactants are also suitable for use in laundry detergent compositions. These surfactants can be broadly described as derivatives of secondary and tertiary amines, derivatives of heterocyclic secondary and tertiary amines, or derivatives of quaternary ammonium, quaternary phosphonium or tertiary sulfonium compounds. See US 3,929,678 (column 19, line 38 through column 22, line 48) for examples of zwitterionic surfactants.
When included therein, the laundry detergent compositions of the present invention typically comprise from 0.2% to about 15%, preferably from about 1% to about 10% by weight of such zwitterionic surfactants.
Semi-polar nonionic surfactants are a special category of nonionic surfactants which include water-soluble amine oxides containing one alkyl moiety of from about 10 to about 18 carbon atoms and 2 moieties selected from the group consisting of alkyl groups and hydroxyalkyl groups containing from about 1 to about 3 carbon atoms; watersoluble phosphine oxides containing one alkyl moiety of from about 10 to about 18 carbon atoms and 2 moieties selected from the group consisting of alkyl groups and hydroxyalkyl groups containing from about 1 to about 3 carbon atoms; and water-soluble sulfoxides containing one alkyl moiety from about 10 to about 18 carbon atoms and a moiety selected from the group consisting of alkyl and hydroxyalkyl moieties of from about 1 to about 3 carbon atoms.
Semi-polar nonionic detergent surfactants include the amine oxide surfactants having the formula:

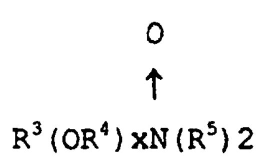

wherein $R^3$ is an alkyl, hydroxyalkyl, or alkyl phenyl group or mixtures thereof containing from about 8 to about 22 carbon atoms; $R^4$ is an alkylene or hydroxyalkylene group containing from about 2 to about 3 carbon atoms or mixtures thereof; x is from 0 to about 3: and each $R^5$ is an alkyl or hydroxyalkyl group containing from about 1 to about 3 carbon atoms or a polyethylene oxide group containing from about 1 to about 3 ethylene oxide groups. The $R^5$ groups can be

attached to each other, e.g., through an oxygen or nitrogen atom, to form a ring structure.

These amine oxide surfactants in particular include $C_{10}$-$C_{18}$ alkyl dimethyl amine oxides and $C_8$-$C_{12}$ alkoxy ethyl dihydroxy ethyl amine oxides.

When included therein, the laundry detergent compositions of the present invention typically comprise from 0.2% to about 15%, preferably from about 1% to about 10% by weight of such semi-polar nonionic surfactants.

**Builder system**

The compositions according to the present invention may further comprise a builder system. Any conventional builder system is suitable for use herein including aluminosilicate materials, silicates, polycarboxylates and fatty acids, materials such as ethylenediamine tetraacetate, metal ion sequestrants such as aminopolyphosphonates, particularly ethylenediamine tetramethylene phosphonic acid and diethylene triamine pentamethylenephosphonic acid. Though less preferred for obvious environmental reasons, phosphate builders can also be used herein.
Suitable builders can be an inorganic ion exchange material, commonly an inorganic hydrated aluminosilicate material, more particularly a hydrated synthetic zeolite such as hydrated zeolite A, X, B, HS or MAP.

Another suitable inorganic builder material is layered silicate, e.g. SKS-6 (Hoechst). SKS-6 is a crystalline layered silicate consisting of sodium silicate ($Na_2Si_2O_5$).

Suitable polycarboxylates containing one carboxy group include lactic acid, glycolic acid and ether derivatives thereof as disclosed in Belgian Patent Nos. 831,368, 821,369 and 821,370. Polycarboxylates containing two carboxy groups include the water-soluble salts of succinic acid, malonic acid, (ethylenedioxy) diacetic acid, maleic acid, diglycollic acid, tartaric acid, tartronic acid and fumaric acid, as well as the ether carboxylates described in German Offenlenschrift 2,446,686, and 2,446,487, US 3,935,257 and the sulfinyl carboxylates described in Belgian Patent No. 840,623. Polycarboxylates containing three carboxy groups include, in particular, water-soluble citrates, aconitrates and citraconates as well as succinate derivatives such as the carboxymethyloxysuccinates described in British Patent No. 1,379,241, lactoxysuccinates described in Netherlands Application 7205873, and the oxypolycarboxylate materials such as 2-oxa-1,1,3-propane tricarboxylates described in British Patent No. 1,387,447.

Polycarboxylates containing four carboxy groups include oxydisuccinates disclosed in British Patent No. 1,261,829, 1,1,2,2,-ethane tetracarboxylates, 1,1,3,3-propane tetracarboxylates containing sulfo substituents include the sulfosuccinate derivatives disclosed in British Patent Nos. 1,398,421 and 1,398,422 and in US 3,936,448, and the sulfonated pyrolysed citrates described in British Patent No. 1,082,179, while polycarboxylates containing phosphone substituents are disclosed in British Patent No. 1,439,000.

Alicyclic and heterocyclic polycarboxylates include cyclopentane-cis,cis-cis-tetracarboxylates, cyclopentadienide pentacarboxylates, 2,3,4,5-tetrahydrofuran - cis, cis, cis-tetracarboxylates, 2,5-tetrahydrofuran-cis, discarboxylates, 2,2,5,5,-tetrahydrofuran - tetracarboxylates, 1,2,3,4,5,6-hexane - hexacarboxylates and carboxymethyl derivatives of polyhydric alcohols such as sorbitol, mannitol and xylitol. Aromatic polycarboxylates include mellitic acid, pyromellitic acid and the phthalic acid derivatives disclosed in British Patent No. 1,425,343.

Of the above, the preferred polycarboxylates are hydroxycarboxylates containing up to three carboxy groups per molecule, more particularly citrates.

Preferred builder systems for use in the present compositions include a mixture of a water-insoluble aluminosilicate builder such as zeolite A or of a layered silicate (SKS-6), and a water-soluble carboxylate chelating agent such as citric acid.

A suitable chelant for inclusion in the detergent composiions in accordance with the invention is ethylenediamine-N,N'-disuccinic acid (EDDS) or the alkali metal, alkaline earth metal, ammonium, or substituted ammonium salts thereof, or mixtures thereof. Preferred EDDS compounds are the free acid form and the sodium or magnesium salt thereof. Examples of such preferred sodium salts of EDDS include $Na_2$EDDS and $Na_4$EDDS. Examples of such preferred magnesium salts of EDDS include MgEDDS and $Mg_2$EDDS. The magnesium salts are the most preferred for inclusion in compositions in accordance with the invention.

Preferred builder systems include a mixture of a water-insoluble aluminosilicate builder such as zeolite A, and a water soluble carboxylate chelating agent such as citric acid.

Other builder materials that can form part of the builder system for use in granular compositions include inorganic materials such as alkali metal carbonates, bicarbonates, silicates, and organic materials such as the organic phosphonates, amino polyalkylene phosphonates and amino polycarboxylates.

Other suitable water-soluble organic salts are the homo- or co-polymeric acids or their salts, in which the polycarboxylic acid comprises at least two carboxyl radicals separated form each other by not more than two carbon atoms.

Polymers of this type are disclosed in GB-A-1,596;756. Examples of such salts are polyacrylates of MW 2000-5000 and their copolymers with maleic anhydride, such copolymers having a molecular weight of from 20,000 to 70,000, especially about 40/000.

Detergency builder salts are normally included in amounts of from 5% to 80% by weight of the composition. Preferred levels of builder for liquid detergents are from 5% to 30%.

**Enzymes**

Preferred detergent compositions, in addition to the enzyme preparation of the invention, comprise other enzyme(s) which provides cleaning performance and/or fabric care benefits.

Such enzymes include proteases, lipases, cutinases, amylases, cellulases, peroxidases, oxidases (e.g. laccases).

Proteases: Any protease suitable for use in alkaline solutions can be used. Suitable proteases include those of animal, vegetable or microbial origin. Microbial origin is preferred. Chemically or genetically modified mutants are included. The protease may be a serine protease, preferably an alkaline microbial protease or a trypsin-like protease. Examples of alkaline proteases are subtilisins, especially those derived from Bacillus, e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the Fusarium protease described in WO 89/06270.

Preferred commercially available protease enzymes include those sold under the trade names Alcalase, Savinase, Primase, Durazym, and Esperase by Novo Nordisk A/S (Denmark), those sold under the tradename Maxatase, Maxacal, Maxapem, Properase, Purafect and Purafect OXP by Genencor International, and those sold under the tradename Opticlean and Optimase by Solvay Enzymes. Protease enzymes may be incorporated into the compositions in accordance with the invention at a level of from 0.00001% to 2% of enzyme protein by weight of the composition, preferably at a level of from 0.0001% to 1% of enzyme protein by weight of the composition, more preferably at a level of from 0.001% to 0.5% of enzyme protein by weight of the composition, even more preferably at a level of from 0.01% to 0.2% of enzyme protein by weight of the composition.

Lipases: Any lipase suitable for use in alkaline solutions can be used. Suitable lipases include those of bacterial or fungal origin. Chemically or genetically modified mutants are included.

Examples of useful lipases include a Humicola lanuginosa lipase, e.g., as described in EP 258 068 and EP 305 216, a Rhizomucor miehei lipase, e.g., as described in EP 238 023, a Candida lipase, such as a C. antarctica lipase, e.g., the C. antarctica lipase A or B described in EP 214 761, a Pseudomonas lipase such as a P. alcaligenes and P. pseudoalcaligenes lipase, e.g., as described in EP 218 272, a P. cepacia lipase, e.g., as described in EP 331 376, a P. stutzeri lipase, e.g., as disclosed in GB 1,372,034, a P. fluorescens lipase, a Bacillus lipase, e.g., a B. subtilis lipase (Dartois et al., (1993), Biochemica et Biophysica acta 1131, 253-260), a B. stearothermophilus lipase (JP 64/744992) and a B. pumilus lipase (WO 91/16422).

Furthermore, a number of cloned lipases may be useful, including the Penicillium camembertii lipase described by Yamaguchi et al., (1991), Gene 103, 61-67), the Geotricum candidum lipase (Schimada, Y. et al., (1989), J. Biochem., 106, 383-388), and various Rhizopus lipases such as a R. delemar lipase (Hass, M.J et al., (1991), Gene 109, 117-113), a R. niveus lipase (Kugimiya et al., (1992), Biosci. Biotech. Biochem. 56, 716-719) and a R. oryzae lipase.

Other types of lipolytic enzymes such as cutinases may also be useful, e.g., a cutinase derived from Pseudomonas mendocina as described in WO 88/09367, or a cutinase derived from Fusarium solani pisi (e.g. described in WO 90/09446).

Especially suitable lipases are lipases such as M1 Lipase™, Luma fast™ and Lipomax™ (Genencor), Lipolase™ and Lipolase Ultra™ (Novo Nordisk A/S), and Lipase P "Amano" (Amano Pharmaceutical Co. Ltd.).

The lipases are normally incorporated in the detergent composition at a level of from 0.00001% to 2% of enzyme protein by weight of the composition, preferably at a level of from 0.0001% to 1% of enzyme protein by weight of the composition, more preferably at a level of from 0.001% to 0.5% of enzyme protein by weight of the composition, even more preferably at a level of from 0.01% to 0.2% of enzyme protein by weight of the composition.

Amylases: Any amylase ($\alpha$ and/or $\beta$) suitable for use in alkaline solutions can be used. Suitable amylases include those of bacterial or fungal origin. Chemically or genetically modified mutants are included. Amylases include, for example, $\alpha$-amylases obtained from a special strain of B. licheniformis, described in more detail in GB 1,296,839. Commercially available amylases are Duramyl™, Termamyl™, Fungamyl™ and BAN™ (available from Novo Nordisk A/S) and Rapidase™ and Maxamyl P™ (available from Genencor).

The amylases are normally incorporated in the detergent composition at a level of from 0.00001% to 2% of enzyme protein by weight of the composition, preferably at a level of from 0.0001% to 1% of enzyme protein by weight of the composition, more preferably at a level of from 0.001% to 0.5% of enzyme protein by weight of the composition, even more preferably at a level of from 0.01% to 0.2% of enzyme protein by weight of the composition.

Cellulases: Any cellulase suitable for use in alkaline solutions can be used. Suitable cellulases include those of bacterial or fungal origin. Chemically or genetically modified mutants are included. Suitable cellulases are disclosed in US 4,435,307, which discloses fungal cellulases produced from Humicola insolens. Especially suitable cellulases are the cellulases having colour care benefits. Examples of such cellulases are cellulases described in European patent

application No. 0 495 257.

Commercially available cellulases include Celluzyme™ produced by a strain of Humicola insolens, (Novo Nordisk A/S), and KAC-500(B)™ (Kao Corporation).

Cellulases are normally incorporated in the detergent composition at a level of from 0.00001% to 2% of enzyme protein by weight of the composition, preferably at a level of from 0.0001% to 1% of enzyme protein by weight of the composition, more preferably at a level of from 0.001% to 0.5% of enzyme protein by weight of the composition, even more preferably at a level of from 0.01% to 0.2% of enzyme protein by weight of the composition.

Peroxidases/Oxidases: Peroxidase enzymes are used in combination with hydrogen peroxide or a source thereof (e.g. a percarbonate, perborate or persulfate). Oxidase enzymes are used in combination with oxygen. Both types of enzymes are used for "solution bleaching", i.e. to prevent transfer of a textile dye from a dyed fabric to another fabric when said fabrics are washed together in a wash liquor, preferably together with an enhancing agent as described in e.g. WO 94/12621 and WO 95/01426. Suitable peroxidases/oxidases include those of plant, bacterial or fungal origin. Chemically or genetically modified mutants are included.

Peroxidase and/or oxidase enzymes are normally incorporated in the detergent composition at a level of from 0.00001% to 2% of enzyme protein by weight of the composition, preferably at a level of from 0.0001% to 1% of enzyme protein by weight of the composition, more preferably at a level of from 0.001% to 0.5% of enzyme protein by weight of the composition, even more preferably at a level of from 0.01% to 0.2% of enzyme protein by weight of the composition.

Mixtures of the above mentioned enzymes are encompassed herein, in particular a mixture of a protease, an amylase, a lipase and/or a cellulase.

The enzyme of the invention, or any other enzyme incorporated in the detergent composition, is normally incorporated in the detergent composition at a level from 0.00001% to 2% of enzyme protein by weight of the composition, preferably at a level from 0.0001% to 1% of enzyme protein by weight of the composition, more preferably at a level from 0.001% to 0.5% of enzyme protein by weight of the composition, even more preferably at a level from 0.01% to 0.2% of enzyme protein by weight of the composition.

Bleaching agents: Additional optional detergent ingredients that can be included in the detergent compositions of the present invention include bleaching agents such as PB1, PB4 and percarbonate with a particle size of 400-800 microns. These bleaching agent components can include one or more oxygen bleaching agents and, depending upon the bleaching agent chosen, one or more bleach activators. When present oxygen bleaching compounds will typically be present at levels of from about 1% to about 25%. In general, bleaching compounds are optional added components in non-liquid formulations, e.g. granular detergents.

The bleaching agent component for use herein can be any of the bleaching agents useful for detergent compositions including oxygen bleaches as well as others known in the art.

The bleaching agent suitable for the present invention can be an activated or non-activated bleaching agent.

One category of oxygen bleaching agent that can be used encompasses percarboxylic acid bleaching agents and salts thereof. Suitable examples of this class of agents include magnesium monoperoxyphthalate hexahydrate, the magnesium salt of meta-chloro perbenzoic acid, 4-nonylamino-4-oxoperoxybutyric acid and diperoxydodecanedioic acid. Such bleaching agents are disclosed in US 4,483,781, US 740,446, EP 0 133 354 and US 4,412,934. Highly preferred bleaching agents also include 6-nonylamino-6-oxoperoxycaproic acid as described in US 4,634,551.

Another category of bleaching agents that can be used encompasses the halogen bleaching agents. Examples of hypohalite bleaching agents, for example, include trichloro isocyanuric acid and the sodium and potassium dichloroisocyanurates and N-chloro and N-bromo alkane sulphonamides. Such materials are normally added at 0.5-10% by weight of the finished product, preferably 1-5% by weight.

The hydrogen peroxide releasing agents can be used in combination with bleach activators such as tetraacetylethylenediamine (TAED), nonanoyloxybenzenesulfonate (NOBS, described in US 4,412,934), 3,5-trimethylhexsanoloxybenzenesulfonate (ISONOBS, described in EP 120 591) or pentaacetylglucose (PAG), which are perhydrolyzed to form a peracid as the active bleaching species, leading to improved bleaching effect. In addition, very suitable are the bleach activators C8(6-octanamido-caproyl) oxybenzene-sulfonate, C9(6-nonanamido caproyl) oxybenzenesulfonate and C10 (6-decanamido caproyl) oxybenzenesulfonate or mixtures thereof. Also suitable activators are acylated citrate esters such as disclosed in European Patent Application No. 91870207.7.

Useful bleaching agents, including peroxyacids and bleaching systems comprising bleach activators and peroxygen bleaching compounds for use in cleaning compositions according to the invention are described in application USSN 08/136,626.

The hydrogen peroxide may also be present by adding an enzymatic system (i.e. an enzyme and a substrate therefore) which is capable of generation of hydrogen peroxide at the beginning or during the washing and/or rinsing process. Such enzymatic systems are disclosed in European Patent Application EP 0 537 381.

Bleaching agents other than oxygen bleaching agents are also known in the art and can be utilized herein. One type of non-oxygen bleaching agent of particular interest includes photoactivated bleaching agents such as the

sulfonated zinc and/or aluminium phthalocyanines. These materials can be deposited upon the substrate during the washing process. Upon irradiation with light, in the presence of oxygen, such as by hanging clothes out to dry in the daylight, the sulfonated zinc phthalocyanine is activated and, consequently, the substrate is bleached. Preferred zinc phthalocyanine and a photoactivated bleaching process are described in US 4,033,718. Typically, detergent composition will contain about 0.025% to about 1.25%, by weight, of sulfonated zinc phthalocyanine.

Bleaching agents may also comprise a manganese catalyst. The manganese catalyst may, e.g., be one of the compounds described in "Efficient manganese catalysts for low-temperature bleaching", Nature 369, 1994, pp. 637-639.

Suds suppressors: Another optional ingredient is a suds suppressor, exemplified by silicones, and silica-silicone mixtures. Silicones can generally be represented by alkylated polysiloxane materials, while silica is normally used in finely divided forms exemplified by silica aerogels and xerogels and hydrophobic silicas of various types. Theses materials can be incorporated as particulates, in which the suds suppressor is advantageously releasably incorporated in a water-soluble or waterdispersible, substantially non surface-active detergent impermeable carrier. Alternatively the suds suppressor can be dissolved or dispersed in a liquid carrier and applied by spraying on to one or more of the other components.

A preferred silicone suds controlling agent is disclosed in US 3,933,672. Other particularly useful suds suppressors are the self-emulsifying silicone suds suppressors, described in German Patent Application DTOS 2,646,126. An example of such a compound is DC-544, commercially available form Dow Corning, which is a siloxane-glycol copolymer. Especially preferred suds controlling agent are the suds suppressor system comprising a mixture of silicone oils and 2-alkyl-alkanols. Suitable 2-alkyl-alkanols are 2-butyl-octanol which are commercially available under the trade name Isofol 12 R.

Such suds suppressor system are described in European Patent Application EP 0 593 841.

Especially preferred silicone suds controlling agents are described in European Patent Application No. 92201649.8. Said compositions can comprise a silicone/ silica mixture in combination with fumed nonporous silica such as Aerosil$^{R}$.

The suds suppressors described above are normally employed at levels of from 0.001% to 2% by weight of the composition, preferably from 0.01% to 1% by weight.

**Other components:** Other components used in detergent compositions may be employed such as soil-suspending agents, soil-releasing agents, optical brighteners, abrasives, bactericides, tarnish inhibitors, coloring agents, and/or encapsulated or nonencapsulated perfumes.

Especially suitable encapsulating materials are water soluble capsules which consist of a matrix of polysaccharide and polyhydroxy compounds such as described in GB 1,464,616.

Other suitable water soluble encapsulating materials comprise dextrins derived from ungelatinized starch acid esters of substituted dicarboxylic acids such as described in US 3,455,838. These acid-ester dextrins are, preferably, prepared from such starches as waxy maize, waxy sorghum, sago, tapioca and potato. Suitable examples of said encapsulation materials include N-Lok manufactured by National Starch. The N-Lok encapsulating material consists of a modified maize starch and glucose. The starch is modified by adding monofunctional substituted groups such as octenyl succinic acid anhydride.

Antiredeposition and soil suspension agents suitable herein include cellulose derivatives such as methylcellulose, carboxymethylcellulose and hydroxyethylcellulose, and homo- or co-polymeric polycarboxylic acids or their salts. Polymers of this type include the polyacrylates and maleic anhydride-acrylic acid copolymers previously mentioned as builders, as well as copolymers of maleic anhydride with ethylene, methylvinyl ether or methacrylic acid, the maleic anhydride constituting at least 20 mole percent of the copolymer. These materials are normally used at levels of from 0.5% to 10% by weight, more preferably form 0.75% to 8%, most preferably from 1% to 6% by weight of the composition.

Preferred optical brighteners are anionic in character, examples of which are disodium 4,4'-bis-(2-diethanolamino-4-anilino -s- triazin-6-ylamino)stilbene-2:2' disulphonate, disodium 4, - 4'-bis-(2-morpholino-4-anilino-s-triazin-6-ylamino-stilbene-2:2' - disulphonate, disodium 4,4' - bis-(2,4-dianilino-s-triazin-6-ylamino)stilbene-2:2' - disulphonate, monosodium 4',4'' - bis-(2,4-dianilino-s-triazin-6 ylamino)stilbene-2-sulphonate, disodium 4,4' -bis-(2-anilino-4-(N-methyl-N-2-hydroxyethylamino)-s-triazin-6-ylamino)stilbene-2,2' - disulphonate, di-sodium 4,4' -bis-(4-phenyl-2,1,3-triazol-2-yl}-stilbene-2,2' disulphonate, di-so-dium 4,4'bis(2-anilino-4-(1-methyl-2-hydroxyethylamino)-s-triazin-6-ylami-no)stilbene-2,2'disulphonate, sodium 2(stilbyl-4''-(naphtho-1',2':4,5)-1,2,3, - triazole-2''-sulphonate and 4,4'-bis(2-sulphostyryl)biphenyl.

Other useful polymeric materials are the polyethylene glycols, particularly those of molecular weight 1000-10000, more particularly 2000 to 8000 and most preferably about 4000. These are used at levels of from 0.20% to 5% more preferably from 0.25% to 2.5% by weight. These polymers and the previously mentioned homo- or co-polymeric polycarboxylate salts are valuable for improving whiteness maintenance, fabric ash deposition, and cleaning performance on clay, proteinaceous and oxidizable soils in the presence of transition metal impurities.

Soil release agents useful in compositions of the present invention are conventionally copolymers or terpolymers of terephthalic acid with ethylene glycol and/or propylene glycol units in various arrangements. Examples of such polymers are disclosed in US 4,116,885 and 4,711,730 and EP 0 272 033. A particular preferred polymer in accordance with EP 0 272 033 has the formula:

$$(CH_3(PEG)_{43})_{0.75}\ (POH)_{0.25}[T\text{-}PO)_{2.8}(T\text{-}PEG)_{0.4}]T(POH)_{0.25}((PEG)_{43}CH_3)_{0.75}$$

where PEG is -($OC_2H_4$)O-, PO is ($OC_3H_6O$) and T is ($pOOC_6H_4CO$).

Also very useful are modified polyesters as random copolymers of dimethyl terephthalate, dimethyl sulfoisophthalate, ethylene glycol and 1,2-propanediol, the end groups consisting primarily of sulphobenzoate and secondarily of mono esters of ethylene glycol and/or 1,2-propanediol. The target is to obtain a polymer capped at both end by sulphobenzoate groups, "primarily", in the present context most of said copolymers herein will be endcapped by sulphobenzoate groups. However, some copolymers will be less than fully capped, and therefore their end groups may consist of monoester of ethylene glycol and/or 1,2-propanediol, thereof consist "secondarily" of such species.

The selected polyesters herein contain about 46%.by weight of dimethyl terephthalic acid, about 16% by weight of 1,2-propanediol, about 10% by weight ethylene glycol, about 13% by weight of dimethyl sulfobenzoic acid and about 15% by weight of sulfoisophthalic acid, and have a molecular weight of about 3.000. The polyesters and their method of preparation are described in detail in EP 311 342.

Softening agents: Fabric softening agents can also be incorporated into laundry detergent compositions in accordance with the present invention. These agents may be inorganic or organic in type. Inorganic softening agents are exemplified by the smectite clays disclosed in GB-A-1 400898 and in US 5,019,292. Organic fabric softening agents include the water insoluble tertiary amines as disclosed in GB-A1 514 276 and EP 0 011 340 and their combination with mono $C_{12}$-$C_{14}$ quaternary ammonium salts are disclosed in EP-B-0 026 528 and di-long-chain amides as disclosed in EP 0 242 919. Other useful organic ingredients of fabric softening systems include high molecular weight polyethylene oxide materials as disclosed in EP 0 299 575 and 0 313 146.

Levels of smectite clay are normally in the range from 5% to 15%, more preferably from 8% to 12% by weight, with the material being added as a dry mixed component to the remainder of the formulation. Organic fabric softening agents such as the water-insoluble tertiary amines or dilong chain amide materials are incorporated at levels of from 0.5% to 5% by weight, normally from 1% to 3% by weight whilst the high molecular weight polyethylene oxide materials and the water soluble cationic materials are added at levels of from 0.1% to 2%, normally from 0.15% to 1.5% by weight. These materials are normally added to the spray dried portion of the composition, although in some instances it may be more convenient to add them as a dry mixed particulate, or spray them as molten liquid on to other solid components of the composition.

Polymeric dye-transfer inhibiting agents: The detergent compositions according to the present invention may also comprise from 0.001% to 10%, preferably from 0.01% to 2%, more preferably form 0.05% to 1% by weight of polymeric dye- transfer inhibiting agents. Said polymeric dye-transfer inhibiting agents are normally incorporated into detergent compositions in order to inhibit the transfer of dyes from colored fabrics onto fabrics washed therewith. These polymers have the ability of compiexing or adsorbing the fugitive dyes washed out of dyed fabrics before the dyes have the opportunity to become attached to other articles in the wash.

Especially suitable polymeric dye-transfer inhibiting agents are polyamine N-oxide polymers, copolymers of N-vinyl-pyrrolidone and N-vinylimidazole, polyvinylpyrrolidone polymers, polyvinyloxazolidones and polyvinylimidazoles or mixtures thereof.

Addition of such polymers also enhances the performance of the enzymes according the invention.

The detergent composition according to the invention can be in liquid, paste, gels, bars or granular forms.
Non-dusting granulates may be produced, e.g., as disclosed in US 4,106,991 and 4,661,452 (both to Novo Industri A/S) and may optionally be coated by methods known in the art. Examples of waxy coating materials are poly(ethylene oxide) products (polyethyleneglycol, PEG) with mean molecular weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591.

Granular compositions according to the present invention can also be in "compact form", i.e. they may have a relatively higher density than conventional granular detergents, i.e. form 550 to 950 g/l; in such case, the granular detergent compositions according to the present invention will contain a lower amount of "Inorganic filler salt", compared to conventional granular detergents; typical filler salts are alkaline earth metal salts of sulphates and chlorides, typically

sodium sulphate; "Compact" detergent typically comprise not more than 10% filler salt. The liquid compositions according to the present invention can also be in "concentrated form", in such case, the liquid detergent compositions according to the present invention will contain a lower amount of water, compared to conventional liquid detergents. Typically, the water content of the concentrated liquid detergent is less than 30%, more preferably less than 20%, most preferably less than 10% by weight of the detergent compositions.

The compositions of the invention may for example, be formulated as hand and machine laundry detergent compositions including laundry additive compositions and compositions suitable for use in the pretreatment of stained fabrics, rinse added fabric softener compositions, and compositions for use in general household hard surface cleaning operations and dishwashing operations.

The following examples are meant to exemplify compositions for the present invention, but are not necessarily meant to limit or otherwise define the scope of the invention.
In the detergent compositions, the abbreviated component identifications have the following meanings:

LAS: Sodium linear $C_{12}$ alkyl benzene sulphonate

TAS: Sodium tallow alkyl sulphate

XYAS: Sodium $C_{1X}$ - $C_{1Y}$ alkyl sulfate

SS: Secondary soap surfactant of formula 2-butyl octanoic acid

25EY: A $C_{12}$ - $C_{15}$ predominantly linear primary alcohol condensed with an average of Y moles of ethylene oxide

45EY: A $C_{14}$ - $C_{15}$ predominantly linear primary alcohol condensed with an average of Y moles of ethylene oxide

XYEZS: $C_{1X}$ - $C_{1Y}$ sodium alkyl sulfate condensed with an average of Z moles of ethylene oxide per mole

Nonionic: $C_{13}$ - $C_{15}$ mixed ethoxylated/propoxylated fatty alcohol with an average degree of ethoxylation of 3.8 and an average degree of propoxylation of 4.5 sold under the tradename Plurafax LF404 by BASF Gmbh

CFAA: $C_{12}$ - $C_{14}$ alkyl N-methyl glucamide

TFAA: $C_{16}$ - $C_{18}$ alkyl N-methyl glucamide

Silicate: Amorphous Sodium Silicate ($SiO_2$:$Na_2O$ ratio = 2.0)

NaSKS-6: Crystalline layered silicate of formula $\delta$-$Na_2Si_2O_5$

Carbonate: Anhydrous sodium carbonate

Phosphate: Sodium tripolyphosphate

MA/AA: Copolymer of 1:4 maleic/acrylic acid, average molecular weight about 80,000

Polyacrylate: Polyacrylate homopolymer with an average molecular weight of 8,000 sold under the tradename PA30 by BASF Gmbh

Zeolite A: Hydrated Sodium Aluminosilicate of formula $Na_{12}(AlO_2SiO_2)_{12}$. $27H_2O$ having a primary particle size in the range from 1 to 10 micrometers

Citrate: Tri-sodium citrate dihydrate

Citric: Citric Acid

Perborate: Anhydrous sodium perborate monohydrate bleach, empirical formula $NaBO_2.H_2O_2$

PB4: Anhydrous sodium perborate tetrahydrate

Percarbonate: Anhydrous sodium percarbonate bleach of empirical formula $2Na_2CO_3.3H_2O_2$

TAED: Tetraacetyl ethylene diamine

CMC: Sodium carboxymethyl cellulose

DETPMP: Diethylene triamine penta (methylene phosphonic acid), marketed by Monsanto under the Tradename Dequest 2060

PVP: Polyvinylpyrrolidone polymer

EDDS: Ethylenediamine-N, N'-disuccinic acid, [S,S] isomer in the form of the sodium salt

Suds Suppressor: 25% paraffin wax Mpt 50°C, 17% hydrophobic silica, 58% paraffin oil

Granular Suds suppressor: 12% Silicone/silica/ 18% stearyl alcohol, 70% starch in granular form

Sulphate: Anhydrous sodium sulphate

HMWPEO: High molecular weight polyethylene oxide

TAE 25: Tallow alcohol ethoxylate (25)

**Detergent Example I**

A granular fabric cleaning composition in accordance with the invention may be prepared as follows:

| | |
|---|---|
| Sodium linear $C_{12}$ alkyl benzene sulfonate | 6.5 |
| Sodium sulfate | 15.0 |
| Zeolite A | 26.0 |
| Sodium nitrilotriacetate | 5.0 |
| Enzyme of the invention | 0.1 |
| PVP | 0.5 |
| TAED | 3.0 |
| Boric acid | 4.0 |
| Perborate | 18.0 |
| Phenol sulphonate | 0.1 |
| Minors | Up to 100 |

**Detergent Example II**

A compact granular fabric cleaning composition (density 800 g/l) in accord with the invention may be prepared as follows:

| | |
|---|---|
| 45AS | 8.0 |
| 25E3S | 2.0 |
| 25E5 | 3.0 |
| 25E3 | 3.0 |
| TFAA | 2.5 |
| Zeolite A | 17.0 |
| NaSKS-6 | 12.0 |
| Citric acid | 3.0 |
| Carbonate | 7.0 |
| MA/AA | 5.0 |
| CMC | 0.4 |

(continued)

| | |
|---|---|
| Enzyme of the invention | 0.1 |
| TAED | 6.0 |
| Percarbonate | 22.0 |
| EDDS | 0.3 |
| Granular suds suppressor | 3.5 |
| water/minors | Up to 100% |

**Detergent Example III**

Granular fabric cleaning compositions in accordance with the invention which are especially useful in the laundering of coloured fabrics were prepared as follows:

| | | |
|---|---|---|
| LAS | 10.7 | - |
| TAS | 2.4 | - |
| TFAA | - | 4.0 |
| 45AS | 3.1 | 10.0 |
| 45E7 | 4.0 | - |
| 25E3S | - | 3.0 |
| 68E11 | 1.8 | - |
| 25E5 | - | 8.0 |
| Citrate | 15.0 | 7.0 |
| Carbonate | - | 10 |
| Citric acid | 2.5 | 3.0 |
| Zeolite A | 32.1 | 25.0 |
| Na-SKS-6 | - | 9.0 |
| MA/AA | 5.0 | 5.0 |
| DETPMP | 0.2 | 0.8 |
| Enzyme of the invention | 0.10 | 0.05 |
| Silicate | 2.5 | - |
| Sulphate | 5.2 | 3.0 |
| PVP | 0.5 | - |
| Poly (4-vinylpyridine)-N-Oxide/copolymer of vinylimidazole and vinylpyrrolidone | - | 0.2 |
| Perborate | 1.0 | - |
| Phenol sulfonate | 0.2 | - |
| Water/Minors | Up to 100% | |

**Detergent Example IV**

Granular fabric cleaning compositions in accordance with the invention which provide "Softening through the wash" capability may be prepared as follows:

| | | |
|---|---|---|
| 45AS | - | 10.0 |
| LAS | 7.6 | - |
| 68AS | 1.3 | - |
| 45E7 | 4.0 | - |
| 25E3 | - | 5.0 |
| Coco-alkyl-dimethyl hydroxyethyl ammonium chloride | 1.4 | 1.0 |
| Citrate | 5.0 | 3.0 |
| Na-SKS-6 | - | 11.0 |
| Zeolite A | 15.0 | 15.0 |
| MA/AA | 4.0 | 4.0 |

(continued)

| | | |
|---|---|---|
| DETPMP | 0.4 | 0.4 |
| Perborate | 15.0 | - |
| Percarbonate | - | 15.0 |
| TAED | 5.0 | 5.0 |
| Smectite clay | 10.0 | 10.0 |
| HMWPEO | - | 0.1 |
| Enzyme of the invention | 0.10 | 0.05 |
| Silicate | 3.0 | 5.0 |
| Carbonate | 10.0 | 10.0 |
| Granular suds suppressor | 1.0 | 4.0 |
| CMC | 0.2 | 0.1 |
| Water/Minors | Up to 100% | |

**Detergent Example V**

Heavy duty liquid fabric cleaning compositions in accordance with the invention may be prepared as follows:

| | I | II |
|---|---|---|
| LAS acid form | - | 25.0 |
| Citric acid | 5.0 | 2.0 |
| 25AS acid form | 8.0 | - |
| 25AE2S acid form | 3.0 | - |
| 25AE7 | 8.0 | - |
| CFAA | 5 | - |
| DETPMP | 1.0 | 1.0 |
| Fatty acid | 8 | - |
| Oleic acid | - | 1.0 |
| Ethanol | 4.0 | 6.0 |
| Propanediol | 2.0 | 6.0 |
| Enzyme of the invention | 0.10 | 0.05 |
| Coco-alkyl dimethyl hydroxy ethyl ammonium chloride | - | 3.0 |
| Smectite clay | - | 5.0 |
| PVP | 2.0 | - |
| Water / Minors | Up to 100% | |

In a preferred embodiment, the detergent or cleaning composition comprises the enhancing agent in an amount effective for killing or inhibiting cells, preferably in an amount above 1 ppm, more preferably above 10 ppm.

When used for preservation of food, beverages, cosmetics such as lotions, creams, gels, ointments, soaps, shampoos, conditioners, antiperspirants, deodorants, mouth wash; contact lens products, enzyme formulations, or food ingredients, the composition used in the method of the present invention may be incorporated into the unpreserved food, beverages, cosmetics, contact lens products, food ingredients or antiinflammatory product in an amount effective for killing or inhibiting growing microbial cells.

Thus, the composition used in the method of the invention may by useful as a disinfectant, e.g in the treatment of acne, infections in the eye or the mouth, skin infections; in antiperspirants or deodorants; in foot bath salts; for cleaning and disinfection of contact lenses, hard surfaces, teeth (oral care), wounds, bruises and the like.

The method of the invention may advantageously be carried out at a relatively high pH, since it is contemplated that the bacteriocidal activities are optimal at high pH values.

In general it is contemplated that the composition of the present invention is useful for cleaning, disinfecting or inhibiting microbial growth on any hard surface. Examples of surfaces, which may advantageously be contacted with the composition of the invention are surfaces of process equipment used e.g. in food processing plants, dairies, chemical or pharmaceutical process plants, water s anitation systems, paper pulp processing plants, water treatment plants, and cooling towers. The composition of the invention should be used in an amount, which is effective for cleaning,

disinfecting or inhibiting microbial growth on the surface in question.

Further, it is contemplated that the composition of the invention can advantageously be used in a cleaning-in-place (C.I.P.) system for cleaning of process equipment of any kind.

The invention is illustrated by the following non-limiting examples.

EXAMPLE 1

**Antibacterial activity of *Coprinus cinereus* recombinant peroxidase using different enhancing agents**

The antibacterial activity of *Coprinus cinereus,* IFO 8371, recombinant peroxidase (rCIP) available from Novo Nordisk A/S, DK-2880 Bagsvaerd, Denmark, has been tested in a phosphate buffer with the following enhancing agents: sodium thiocyanate (NaSCN), potassium iodide (KI), 10-phenothiazine propionic acid (PPT), butyl syringate (BS) and 2,2' azinobis(3-ethylbenzothiazoline-6-sulfonate) (ABTS). The hydrogen peroxide was either generated by glucose oxidase or added directly in the concentration of 5 mM. In order to avoid interference with substrate components, the experiment was carried out in a buffer instead of in a growth substrate.

*P. fluorescens* ($10^4$ cfu/ml) was treated with rCIP (3 POXU/ml) and the different enhancing agents (5 mM) for 15 min (pH 6.0, 40°C) combined with glucoseoxidase/glucose or hydrogen peroxide, respectively. The bactericidal activity was determined by plate counting and by incubation in Malthus. The detection times measured by the Malthus instrument were converted to cfu/ml by a standard curve.

Indirect Malthus measurements were used when enumerating total survival cells (Malthus Flexi M2060, Malthus Instrument Limited). 3 ml of growth medium was transferred to the outer chamber of the indirect Malthus cells, and 0.5 ml of sterile KOH (0.1 M) was transferred to the inner chamber. The cell suspensions were after enzyme treatment transferred to the outer chamber of the Malthus cell. As cells are growing in the outer chamber they produce $CO_2$ which will dissolve in the KOH in the inner chamber and thereby change the conductance of the KOH. The amount of $CO_2$ formed by the respiring cells surviving the enzyme treatment was used for estimating the number of viable cells. When the conductance change is measurable by the Malthus, a detection time (dt) will be recorded. The dt's were converted to colony counts by use of a calibration curve relating cfu/ml to dt (fig. 1).

The results are shown in fig. 1 as the number of cells which survive the treatment (average from both Malthus experiments and plate counts). Glucose oxidase and hydrogen peroxide in the used concentration have a bactericidal activity, decreasing the cell number from $10^4$ cfu/ml to approximately $10^2$-$10^3$ cfu/ml, independent of enhancing agent.

rCIP had a statistical significant bactericidal activity against *P. fluorescens* when NaSCN or KI were used as enhancing agents. PPT had a slight bactericidal activity, however, only statistically significant at a 10% level. When KI was used as enhancing agent the activity was 100% bactericidal, both when determined by plate counts and Malthus.

EXAMPLE 2

**Antibacterial activity of rCIP and KI against *P. aeruginosa* and *S. aureus.***

The antibacterial activity of rCIP was tested in phosphate buffer (pH 6.0) against *Pseudomonas aeruginosa* ATCC 10146 and *Staphylococcus aureus* ATCC 25923 with potassium iodide as electron donor, and hydrogen peroxide was added as electron acceptor. The cells (approximately $10^7$ cfu/ml) were incubated with enzyme for 15 min at 40°C, and the experiment was carried out using a $2^3$ factorial design reproduced two times. The bactericidal activity was determined by incubation in Malthus. The detection times measured by the Malthus instrument were converted to cfu/ml by a standard curve.

| Results for *P. aeruginosa:* | | | |
|---|---|---|---|
| rCIP (POXU/ml) | $H_2O_2$ (mM) | KI (mM) | survivors (cfu/ml) |
| 0 | 0 | 0 | $1.7*10^7$ |
| 0 | 0 | 0.5 | $2.1*10^7$ |
| 0 | 0.5 | 0 | $1.2*10^7$ |
| 0 | 0.5 | 0.5 | $6.6*10^6$ |
| 0.5 | 0 | 0 | $2.6*10^7$ |
| 0.5 | 0 | 0.5 | $1.7*10^7$ |
| 0.5 | 0.5 | 0 | $9.0*10^6$ |
| 0.5 | 0.5 | 0.5 | $1.0*10^5$ |

The combination of rCIP with KI and hydrogen peroxide killed 99.4% of *P. aeruginosa,* which is a significant effect of the peroxidase compared to the combination of hydrogen peroxide and KI without peroxidase.

| Results for *S. aureus:* | | | |
|---|---|---|---|
| rCIP (POXU/ml) | $H_2O_2$ (mM) | KI (mM) | survivors (cfu/ml) |
| 0 | 0 | 0 | 2.9*108 |
| 0 | 0 | 0.5 | $2.0*10^8$ |
| 0 | 0.5 | 0 | $1.3*10^8$ |
| 0 | 0.5 | 0.5 | $1.4*10^8$ |
| 0.5 | 0 | 0 | $1.6*10^8$ |
| 0.5 | 0 | 0.5 | $3.4*10^8$ |
| 0.5 | 0.5 | 0 | $1.8*10^8$ |
| 0.5 | 0.5 | 0.5 | $5.6*10^6$ |

The bactericidal activity against *S. aureus* was significant for the combination of peroxidase, hydrogen peroxide and potassium iodide, whereas no activity of hydrogen peroxide combined with iodide was observed. The activity was observed as a 98% reduction in cfu/ml.

Increasing the concentration of peroxidase to 1 POXU/ml and the concentration of hydrogen peroxide and potassium iodide to 1 mM caused a total kill of the cell suspension.

## Claims

1. An enzymatic antimicrobial composition comprising

   a) a peroxidase derivable from the fungus *Coprinus,*
   b) an electron donor selected from a water-soluble halide and thiocyanate salt, and
   c) hydrogen peroxide or a source of hydrogen peroxide.

2. The composition according to claim 1, wherein the peroxidase is a recombinant enzyme obtainable from *Coprinus cinereus.*

3. The composition according to claim 1 or 2, wherein the peroxidase is obtainable from *Coprinus cinereus,* IFO 8371, or is immunologically cross-reactive with the peroxidase obtainable from *Coprinus cinereus,* IFO 8371.

4. The composition according to any of the claims 1-3, wherein the source of hydrogen peroxide is an enzymatic hydrogen peroxide-generating system.

5. The composition according to claim 4, wherein the enzymatic system is selected from the group consisting of glucose oxidase/glucose, hexose oxidase/hexose, L- or D-amino acid oxidase/L- or D-amino acid, and lactate oxidase/lactate.

6. A method of killing or inhibiting a microorganism wherein said microorganism is contacted with a composition comprising:

a) a peroxidase derivable from the fungus Coprinus,
b) an enhancing agent of the formula:

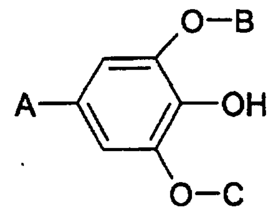

wherein A denotes a group -D, -CH=CH-D, -CH=CH-CH=CH-D, -CH=N-D, -N=N-D, or -N=CH-D; D is selected from the group consisting of -CO-E, $-SO_2$-E, -N-XY, and $-N^+$-XYZ; E is -H, -OH, -R, or -OR, and X and Y and Z may be identical or different and selected from -H and -R; R is $C_1$-$C_{16}$ alkyl, preferably saturated or unsaturated, branched or unbranched $C_1$-$C_8$ alkyl, optionally substituted with a carboxy, sulfo or amino group; and B and C may be the same or different and selected from $C_mH_{2m+1}$; $1 \leq m \leq 5$,
or a water-soluble halide and thiocyanate salt, and
c) hydrogen peroxide or a source of hydrogen peroxide.

7. A method of inhibiting microorganisms present in laundry, wherein the laundry is treated with a soaking, washing or rinsing liquor comprising an effective amount of the composition according to any of the claims 1-5 or the composition used in the method of claim 6.

8. The method according to claim 7, wherein the laundry is treated in a washing machine.

9. The composition of claims 1-5 or the composition used in claim 6 for killing microbial cells present on human or animal skin, hair, oral cavity, mucous membranes, teeth, wounds, bruises or in the eye or inhibiting the growth thereof.

10. The composition according to claim 9, wherein the composition is a liquid composition having disinfectant or preserving properties such as a mouth wash composition, an antiinflammatory liquid (a disinfectant), an eye lotion, a antiperspirant, a deodorant, and a nasal spray; or a solid composition having disinfectant or preserving properties such as an eye ointment, an anti-inflammatory ointment or cream, a foot bath salt, a antiperspirant, and a deodorant.

11. A method of preserving a cosmetic product, wherein an effective amount of the composition according to any of the claims 1-5 or the composition used in the method of claim 6 is incorporated into the cosmetic product.

12. The method according to claim 11, wherein the cosmetic product is a mouth wash composition, a cosmetic liquid or gel or paste, an eye lotion, a antiperspirant, a deodorant, a nasal spray, an eye ointment, an ointment or cream, a foot bath salt.

13. Use of the composition according to any of the claims 1-5 or the composition used in the method of claim 6 for cleaning or disinfection of contact lenses.

14. A method of cleaning, disinfecting or inhibiting microbial growth on a hard surface, wherein the surface is contacted with the composition according to any of the claims 1-5 or the composition used in the method of claim 6.

15. The method according to claim 14, wherein the hard surface is a process equipment member of a cooling tower, a water treatment plant, a dairy, a food processing plant, a chemical or pharmaceutical process plant.

16. The method according to claim 14, wherein the hard surface is a surface of water sanitation equipment.

17. The method according to claim 14, wherein the hard surface is a surface of equipment for paper pulp processing.

18. Use of the composition according to any of the claims 1-5 or the composition used in the method of claim 6 in a

cleaning-in-place (C-I-P) system.

**Patentansprüche**

**1.** Enzymatische antimikrobielle Zusammensetzung enthaltend

a) eine Peroxidase gewinnbar aus dem Pilz *Coprinus*,
b) einen Elektronendonor ausgewählt aus einem wasserlöslichen Haloid und Thiozyanatsalz und
c) Wasserstoffperoxid oder eine Quelle von Wasserstoffperoxid.

**2.** Zusammensetzung nach Anspruch 1, wobei die Peroxidase ein rekombinantes Enzym erhältlich aus *Coprinus cinereus* ist.

**3.** Zusammensetzung nach Anspruch 1 oder 2, wobei die Peroxidase erhältlich ist aus *Coprinus cinereus*, IFO 8371 oder immunologisch kreuzreagierend ist mit der Peroxidase erhältlich aus *Coprinus cinereus*, IFO 8371.

**4.** Zusammensetzung nach einem der Ansprüche 1-3, wobei die Wasserstoffperoxidquelle ein enzymatisches Wasserstoffperoxid-generierendes System ist.

**5.** Zusammensetzung nach Anspruch 4, wobei das enzymatische System ausgewählt ist aus der Gruppe bestehend aus Glukoseoxidase/Glukose, Hexoseoxidase/Hexose, L- oder D-Aminosäureoxidase/L- oder D-Aminosäure und Laktatoxidase/Laktat.

**6.** Verfahren zur Abtötung oder Inhibierung von Mikroorganismen, wobei der Mikroorganismus mit einer Zusammensetzung in Kontakt gebracht wird, enthaltend:

a) eine Peroxidase erhältlich von dem Pilz *Coprinus*,
b) ein Verstärkungsmittel der Formel:

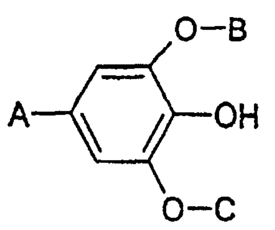

wobei A eine Gruppe -D, -CH=CH-D, -CH=CH-CH=CH-D, -CH=N-D, - N=N-D oder -N=CH-D bezeichnet; D ausgewählt ist aus der Gruppe bestehend aus -CO-E, $-SO_2$-E, -N-XY und $-N^+$-XYZ; E ist -H, -OH, -R oder -OR und X und Y und Z können identisch oder unterschiedlich und ausgewählt sein aus -H und -R; R ist $C_1$-$C_{16}$ Alkyl, vorzugsweise gesättigtes oder ungesättigtes, verzweigtes oder unverzweigtes $C_1$-$C_8$ Alkyl, gegebenenfalls substituiert mit einer Carboxy-, Sulpho- oder Aminogruppe; und B und C können gleich oder unterschiedlich sein und ausgewählt sein aus $C_mH_{2m+1}$; $1 \leq m \leq 5$ oder ein wasserlösliches Haloid und Thiozyanatsalz und
c) Wasserstoffperoxid oder eine Quelle von Wasserstoffperoxid.

**7.** Verfahren zur Inhibition von Mikroorganismen vorhanden in Wäsche, wobei die Wäsche mit einer Einweich-, Wasch- oder Spülflüssigkeit enthaltend eine wirksame Menge der Zusammensetzung nach einem der Ansprüche 1-5 oder enthaltend die Zusammensetzung verwendet in dem Verfahren nach Anspruch 6, behandelt wird.

**8.** Verfahren nach Anspruch 7, wobei die Wäsche in einer Waschmaschine behandelt wird.

**9.** Zusammensetzung der Ansprüche 1-5 oder die Zusammensetzung in Anspruch 6, verwendet zur Abtötung von Mikroben-Zellen, vorhanden auf menschlicher oder tierischer Haut, Haar, Mundhöhle, Schleimhäuten, Zähnen, Wunden, Blutergüssen oder im Auge oder Hemmung ihres Wachstums.

**10.** Zusammensetzung nach Anspruch 9, wobei die Zusammensetzung eine flüssige Zusammensetzung mit Desinfektions- oder Konservierungseigenschaften ist, wie z.B. eine Mundwäschezusammensetzung, eine antiinflammatorische Flüssigkeit (ein Desinfektionsmittel), eine Augenlösung, ein Schwitzmittel, eine Deodorant und ein Nasenspray; oder eine feste Zusammensetzung mit Desinfektions- oder Konservierungseigenschaften, wie z.B. ein Augenöl, eine antiinflammatorisches Öl oder Creme, ein Fußbadsalz, ein Schwitzmittel und ein Deodorant.

**11.** Verfahren zur Konservierung eines kosmetischen Produktes, wobei eine wirksame Menge der Zusammensetzung nach einem Ansprüche 1-5 oder die Zusammensetzung verwendet in dem Verfahren nach Anspruch 6 in das kosmetische Produkt eingeschlossen ist.

**12.** Verfahren nach Anspruch 11, wobei das kosmetische Produkt eine Mundwäschezusammensetzung, eine kosmetische Flüssigkeit oder Gel oder Paste, eine Augenlotion, eine Schwitzmittel, ein Deodorant, eine Nasenspray, ein Augenöl, ein Öl oder Creme, ein Fußbadesalz ist.

**13.** Verwendung der Zusammensetzung nach einem der Ansprüche 1-5 oder Zusammensetzung verwendet in dem Verfahren nach Anspruch 6 zur Reinigung oder Desinfektion von Kontaktlinsen.

**14.** Verfahren zur Reinigung, Desinfektion oder Inhibition mikrobiellen Wachstums auf einer harten Oberfläche, wobei die Oberfläche mit der Zusammensetzung nach einem der Ansprüche 1-5 oder der Zusammensetzung verwendet in dem Verfahren nach Anspruch 6 in Kontakt gebracht wird.

**15.** Verfahren nach Anspruch 14, wobei die harte Oberfläche ein Verfahrensgerätschaftsteil eines Kühlturms, einer Wasseraufbereitungsanlage, einer Molkerei, einer Nahrungsmittel verarbeitenden Anlage, einer chemischen oder einer pharmazeutischen Fertigungsanlage ist.

**16.** Verfahren nach Anspruch 14, wobei die harte Oberfläche die Oberfläche von Wasser-Reinigungsgerätschaften ist.

**17.** Verfahren nach Anspruch 14, wobei die harte Oberfläche eine Oberfläche von Gerätschaften für Papierzellstoffverarbeitung ist.

**18.** Verwendung der Zusammensetzung nach einem der Ansprüche 1 bis 5 oder der Zusammensetzung verwendet in dem Verfahren nach Anspruch 6 in einem An-Ort-Und-Stelle-Reinigungs- (cleaning-in-place, C-I-P) System.

## Revendications

**1.** Composition antimicrobienne enzymatique comprenant

a) une peroxydase pouvant provenir du champignon *Coprinus*,
b) un donneur d'électrons choisi parmi un halogénure soluble dans l'eau et un sel thiocyanate, et
c) du peroxyde d'hydrogène ou une source de peroxyde d'hydrogène.

**2.** Composition selon la revendication 1, dans laquelle la peroxydase est une enzyme recombinante pouvant être obtenue à partir de *Coprinus cinereus.*

**3.** Composition selon la revendication 1 ou 2, dans laquelle la peroxydase peut être obtenue à partir de *Coprinus cinereus*, IFO 8371, ou présente une réactivité croisée immunologique avec la peroxydase pouvant être obtenue à partir de *Coprinus cinereus,* IFO 8371.

**4.** Composition selon l'une quelconque des revendications 1 à 3, dans laquelle la source de peroxyde d'hydrogène est un système enzymatique générateur de peroxyde d'hydrogène.

**5.** Composition selon la revendication 4, dans laquelle le système enzymatique est choisi dans l'ensemble comprenant les systèmes glucose-oxydase/glucose, hexose-oxydase/hexose, acide L- ou D-aminé-oxydase/acide L- ou D-aminé, ou lactate-oxydase/lactate.

**6.** Procédé de destruction ou d'inhibition d'un microorganisme, dans lequel ledit microorganisme est mis en contact avec une composition comprenant :

a) une peroxydase pouvant être obtenue à partir du champignon *Coprinus,*
b) un agent renforçateur de formule :

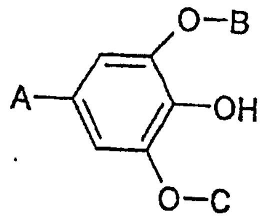

dans laquelle A désigne un groupe -D, -CH=CH-D, -CH=CH-CH=CH-D, -CH=N-D, -N=N-D ou -N=CH-D ; D est choisi dans l'ensemble comprenant -CO-E, $-SO_2$-E, -N-XY et $-N^+$-XYZ ; E est -H, -OH, -R ou -OR, et X et Y et Z peuvent être identiques ou différents et sont choisis parmi -H et -R ; R est un groupe alkyle en $C_1$-$C_{16}$, de préférence un groupe alkyle en $C_1$-$C_8$ saturé ou insaturé à chaîne droite ou ramifiée, éventuellement substitué par un groupe carboxy, sulfo ou amino ; et B et C peuvent être identiques ou différents et sont choisis parmi les radicaux $C_mH_{2m+1}$ ; $1 \leq m \leq 5$,
ou un halogénure soluble dans l'eau et un sel thiocyanate, et
c) du peroxyde d'hydrogène ou une source de peroxyde d'hydrogène.

**7.** Procédé d'inhibition de microorganismes présents dans du linge, dans lequel le linge est traité avec un bain d'imprégnation, de lavage ou de rinçage, comprenant une quantité efficace de la composition selon l'une quelconque des revendications 1 à 5 ou de la composition utilisée dans le procédé de la revendication 6.

**8.** Procédé selon la revendication 7, dans lequel le linge est traité dans une machine à laver.

**9.** Composition selon les revendications 1 à 5, ou composition utilisée dans la revendication 6, pour détruire des cellules microbiennes présentes sur la peau, les cheveux, la cavité buccale, les membranes muqueuses, les dents, les blessures, les ecchymoses ou dans l'oeil d'un sujet humain ou animal, ou pour inhiber leur prolifération.

**10.** Composition selon la revendication 9, dans laquelle la composition est une composition liquide ayant des propriétés désinfectantes ou conservatrices, telles qu'une composition de bain de bouche, un liquide anti-inflammatoire (un désinfectant), une lotion oculaire, un agent anti-transpirant un désodorisant et une pulvérisation nasale ; ou une composition solide ayant des propriétés désinfectantes ou conservatrices telles qu'une pommade pour les yeux, une pommade ou une crème anti-inflammatoire, un sel de bain pour les pieds, un agent anti-transpirant et un désodorisant.

**11.** Procédé de conservation d'un produit cosmétique, dans lequel une quantité efficace de la composition selon l'une quelconque des revendications 1 à 5 ou de la composition utilisée dans le procédé de la revendication 6 est incorporée dans le produit cosmétique.

**12.** Procédé selon la revendication 11, dans lequel le produit cosmétique est une composition de bain de bouche, un liquide ou un gel ou une pâte cosmétique, une lotion pour les yeux, un agent anti-transpirant, un désodorisant, une pulvérisation nasale, une pommade pour les yeux, une pommade ou une crème, un sel de bain pour les pieds.

**13.** Utilisation de la composition selon l'une quelconque des revendications 1 à 5 ou de la composition utilisée dans le procédé de la revendication 6 pour nettoyer ou désinfecter des lentilles de contact.

**14.** Procédé de nettoyage, de désinfection ou d'inhibition de la prolifération microbienne sur une surface dure, dans lequel la surface est mise en contact avec la composition selon l'une quelconque des revendications 1 à 5 ou la composition utilisée dans le procédé de la revendication 6.

**15.** Procédé selon la revendication 14, dans lequel la surface dure est un élément d'équipement de procédé d'une tour de refroidissement, d'une installation de traitement des eaux, d'une laiterie, d'une usine de transformation de produits alimentaires, d'une usine de traitement de produits chimiques ou pharmaceutiques.

**16.** Procédé selon la revendication 14, dans lequel la surface dure est une surface d'un équipement pour améliorer l'état sanitaire de l'eau.

**17.** Procédé selon la revendication 14, dans lequel la surface dure est une surface d'un équipement pour le traitement de la pâte à papier.

**18.** Utilisation de la composition selon l'une quelconque des revendications 1 à 5 ou de la composition utilisée dans le procédé de la revendication 6 dans un système de nettoyage en place (cleaning-in-place, C-I-P).

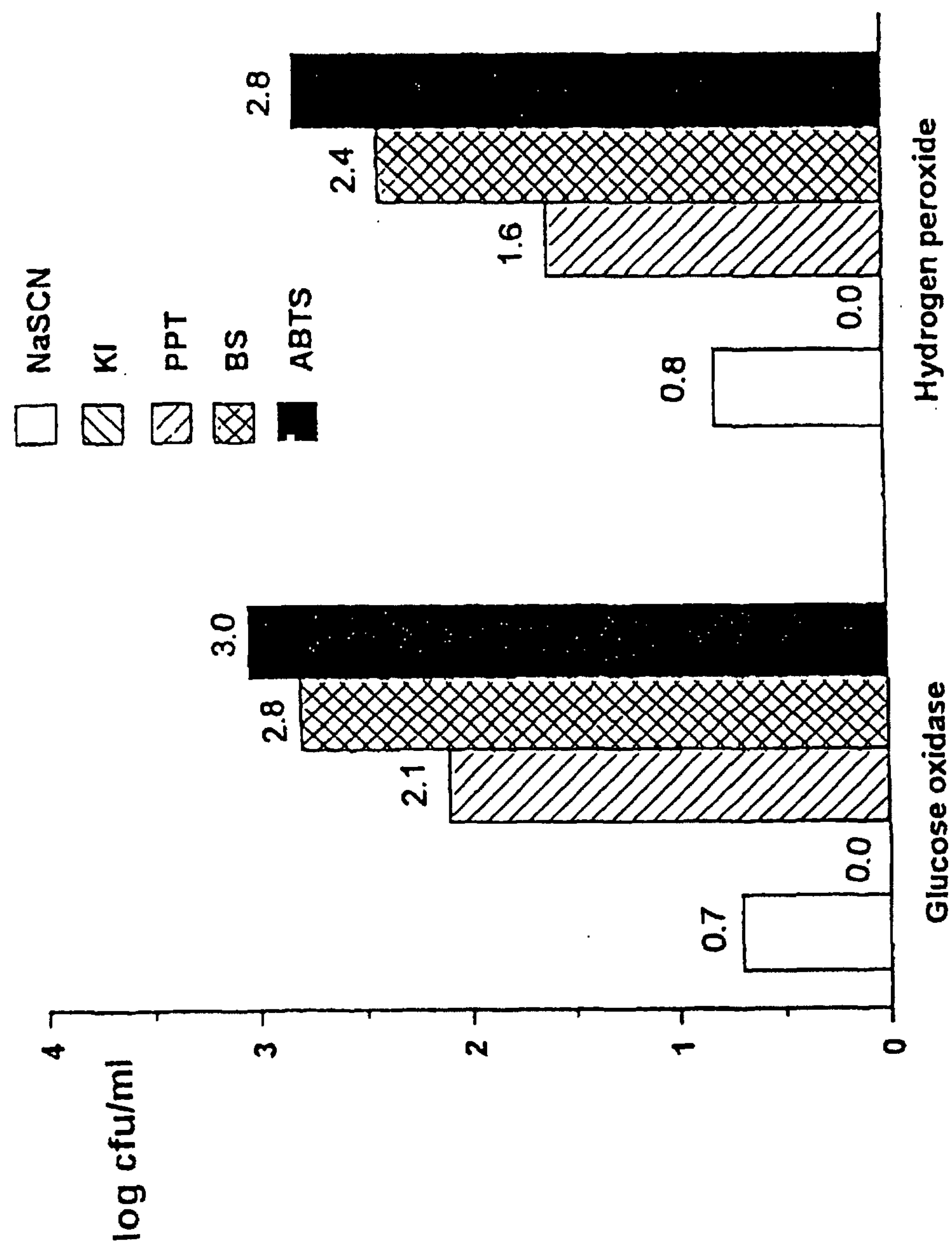
log cfu/ml
4
3
2
1
0
NaSCN
KI
PPT
BS
ABTS
0.7
0.0
2.1
2.8
3.0
Glucose oxidase
0.8
0.0
1.6
2.4
2.8
Hydrogen peroxide

Fig. 1